# EUROPEAN PATENT APPLICATION

(11) **EP 4 335 868 A1**
(43) Date of publication of application: **13.03.2024**
(21) Application number: 22798660.1
(22) Date of filing: 06.05.2022
(51) Int. Cl.: C07K 16/00, C12N 9/16, A61K 39/00

(54) **FC MUTANT WITH ALTERED BINDING TO FC RECEPTOR**

(30) Priority: 07.05.2021 CN 202110495363; 25.04.2022 CN 202210436873
(71) Applicant: Innovent Biologics (Suzhou) Co., Ltd., Suzhou, Jiangsu 215123 (CN)
(72) Inventor: FU, Fenggen, Suzhou, Jiangsu 215123 (CN); ZHOU, Shuaixiang, Suzhou, Jiangsu 215123 (CN); WU, Zhihai, Suzhou, Jiangsu 215123 (CN)
(74) Representative: Sagittarius IP
(86) International application number: PCT/CN2022/091187
(87) International publication number: WO 2022/233320

(57) **Abstract**

The present invention relates to a modified immunoglobulin constant region (Fc) mutant molecule having reduced binding to FcyR or C1q. The molecule can significantly reduce undesired ADCC and/or ADCP and/or CDC effector function *in vivo.* In addition, the present invention relates to a use of the Fc mutant molecule, a fusion protein comprising the mutant molecule and a use thereof.

## Description

### TECHNICAL FIELD

The present invention generally relates to the field of immunology and antibody engineering. Specifically, the present invention relates to a variant of IgG immunoglobulins, a preparation method therefor, and use thereof.

### BACKGROUND

With the development of therapeutic antibodies in recent years, in one aspect, researchers continue to search for new targets, and in another aspect, they continue to upgrade and engineer druggable antibodies in efforts to improve or enhance the beneficial effects. One of the focuses of research is on engineering of the Fc region of an antibody.

It is well known that although the Fc region of an antibody has no antigen-binding activity, it is the site of interaction of an antibody with cell surface Fc receptors and thus plays a critical role in the effector functions of an antibody. The Fc region allows the antibody to exert a variety of effector functions such as antibody-dependent cell-mediated cytotoxicity (ADCC), antibody-dependent cellular phagocytosis (ADCP), and complement-dependent cytotoxicity (CDC) by interacting with different Fc receptors. With respect to ADCC, ADCP, and CDC effector functions, effective or even enhanced ADCC, ADCP, and CDC effector functions are beneficial for the therapeutic effect of an antibody for recognizing an antigen expressed on a tumor cell or pathogen and targeting the elimination of malignant cells, whereas reduced or even eliminated ADCC, ADCP, and CDC effector functions are often required for antibodies targeting, for example, an antigen expressed on a T cell to block cell surface receptors/cytokines or immunomodulation, as antibodies targeting cell surface antigens may induce unnecessary immune stimulation to immune cells and associated effector functions and complement activation with negative consequences, which is particularly important for immune checkpoint inhibitor antibodies requiring low loss of the target cell.

Human IgG subclasses (IgGl, IgG2, IgG3, and IgG4) have different immune functions. For example, IgG antibodies of different subclasses have different ADCC activities, and IgG1 and IgG3 have stronger ADCC activities compared to other subclasses; IgGl, IgG2, IgG3, and IgG4 have antibody-dependent cellular phagocytosis (ADCP). Since a fusion construct of therapeutic antibodies with Fc often requires targeting and activation or neutralization of target ligand functional region, but can not destroy or damage the desired local cells or tissues, there is a continuing real need for Fc mutants with reduced or eliminated Fc effector functions (e.g., reduced or eliminated ADCC and/or ADCP activity, and reduced or eliminated CDC activity) in actual production and applications, and the present application satisfies the need.

It has been reported in the literature that mutation of a part of amino acids in the Fc region may reduce or eliminate ADCC and/or ADCP activity and reduce or eliminate CDC activity. For example, patent families US8734791B2, US10183999B2, US8883147B2 of XENCOR, patent family CN103476795B of Roche, and the like, mostly can reduce ADCC and CDC activity, however, mutations that can completely eliminate ADCC and CDC effects are rare. The present application obtains Fc mutant molecules that can completely eliminate ADCC and CDC effects by directional engineering of wild-type Fc amino acid sequences.

### SUMMARY

The present invention provides a modified mutant molecule of an immunoglobulin constant region (Fc region) that can be used to engineer an antibody or antibody therapeutic agent.

By including the mutation and/or combination thereof disclosed herein in the Fc region, the binding of antibodies and antibody therapeutic agents comprising the mutant Fc region of the present application and other molecules comprising a mutant Fc region to FcyR or C1q is greatly reduced compared to molecules comprising a wild-type Fc region, thereby significantly reducing undesirable ADCC and/or ADCP and/or CDC effector functions *in vivo.* Further, the Fc mutation disclosed herein does not affect the binding ability of antibodies and antibody therapeutic agents comprising the mutant Fc region of the present application and other molecules comprising a mutant Fc region to FcRn, and thus does not affect the half-life of the corresponding molecule. Accordingly, the present application provides an Fc region polypeptide molecule carrying a specific mutation and an antibody molecule comprising the mutant Fc region described above that has a reduced or eliminated ADCC and/or ADCP and/or CDC effector function but still retains the ability to bind to FcRn, or a molecule having a similar structure.

In a first aspect, the present application provides an Fc mutant having a different modification that exhibit reduced affinity for an Fc receptor and/or C1q compared to a wild-type Fc region, thereby reducing or eliminating ADCC, CDC, and ADCP effector functions induced by the Fc mutant. In one embodiment, the ADCC, CDC, and ADCP effector functions induced by the Fc mutant are reduced to at least 80%, 70%, 60%, 50%, 40%, 30%, 20%, 10%, 5%, 2%, or 1% of the ADCC, CDC, and ADCP effector functions induced by a corresponding wild-type Fc, or completely eliminated.

In one embodiment, the Fc mutant provided herein comprises one or more amino acid deletions. In one specific embodiment, the Fc mutant provided herein comprises a deletion at amino acid position 329 (Δ329) according to EU index numbering as in Kabat. In one specific embodiment, the Fc mutant provided herein differs from the corresponding wild-type Fc by the deletion at position 329 (Δ329) according to EU index numbering as in Kabat. In another specific embodiment, the Fc mutant provided herein comprises a deletion at amino acid positions 329 and 330 (Δ329 and Δ330) according to EU index numbering as in Kabat. In one specific embodiment, the Fc mutant provided herein differs from the corresponding wild-type Fc by the deletion at amino acid positions 329 and 330 (Δ329 and Δ330) according to EU index numbering as in Kabat. In one embodiment, the Fc mutant provided herein comprises one or more amino acid substitutions. In one specific embodiment, the Fc mutant provided herein comprises a substitution at amino acid positions 234 and/or 235 according to EU index numbering as in Kabat, wherein L, V, and F at position 234 and/or L at position 235 are substituted by A, V, L, and I, respectively. In one preferred embodiment, the Fc mutant comprises L234A + L235A, V234A, or F234A + L235A. In one specific embodiment, the Fc mutant provided herein differs from the corresponding wild-type Fc by L234A + L235A, V234A, or F234A + L235A.

In one embodiment, the Fc mutant provided herein comprises one or more amino acid deletions and one or more amino acid substitutions. In one specific embodiment, the Fc mutant provided herein comprises a deletion at amino acid position 329 and a substitution at position 330 according to EU index numbering as in Kabat, wherein position 330 is substituted by G, D, or Q. In one preferred embodiment, the Fc mutant comprises Δ329 + A330G or Δ329 + S330G. In one specific embodiment, the Fc mutant provided herein differs from the corresponding wild-type Fc by Δ329 + A330G or Δ329 + S330G.

In one specific embodiment, the Fc mutant provided herein comprises a deletion at amino acid position 329 and a substitution at amino acid positions 234 and 235 according to EU index numbering as in Kabat, wherein L, V, and F at position 234 and/or L at position 235 are substituted by A, V, L, and I, respectively. In one preferred embodiment, the Fc mutant comprises L234A + L235A + Δ329, V234A + Δ329, or F234A + L235A + Δ329. In one specific embodiment, the Fc mutant provided herein differs from the corresponding wild-type Fc by L234A + L235A + Δ329, V234A + Δ329, or F234A + L235A + Δ329.

In another specific embodiment, the Fc mutant provided herein comprises a deletion at amino acid positions 329 and 330 and a substitution at amino acid positions 234 and 235 according to EU index numbering as in Kabat, wherein L, V, and F at position 234 and/or L at position 235 are substituted by A, V, L, and I, respectively. In one preferred embodiment, the Fc mutant comprises L234A + L235A + Δ329 + Δ330, V234A + Δ329 + Δ330, or F234A + L235A + Δ329 + Δ330. In one specific embodiment, the Fc mutant provided herein differs from the corresponding wild-type Fc by L234A + L235A + Δ329 + Δ330, V234A + Δ329 + Δ330, or F234A + L235A + Δ329 + Δ330.

In another specific embodiment, the Fc mutant provided herein comprises a deletion at amino acid position 329 and a substitution at amino acid positions 234, 235, and 330 according to EU index numbering as in Kabat, wherein L, V, and F at position 234 and/or L at position 235 are substituted by A, V, L, and I, respectively, and position 330 is substituted by G, D, or Q. In one preferred embodiment, the Fc mutant comprises L234A + L235A + A330G + Δ329, L234A + L235A + S330G + Δ329, V234A + L235A + A330G + Δ329, V234A + L235A + S330G + Δ329, F234A + L235A + S330G + Δ329, or F234A + L235A + A330G + Δ329. In one specific embodiment, the Fc mutant provided herein differs from the corresponding wild-type Fc by L234A + L235A + A330G + Δ329, L234A + L235A + S330G + Δ329, V234A + L235A + A330G + Δ329, V234A + L235A + S330G + Δ329, F234A + L235A + S330G + Δ329, or F234A + L235A + A330G + Δ329. In some embodiments, the Fc mutant provided herein is an IgG1 Fc mutant, and the mutant has reduced or even eliminated ability to bind to FcyR compared to the corresponding wild-type Fc region. In other embodiments, the Fc mutant provided herein is an IgG2, IgG3, or IgG4 Fc mutant, and the mutant has reduced or even eliminated ability to bind to FcyR compared to the corresponding wild-type Fc region. Preferably, the Fc mutant provided herein retains the ability to bind to FcRn.

The Fc mutant disclosed in the present application can be used as a platform component in any scenario where ADCC/ADCP/CDC effector function needs to be reduced or even eliminated, for example, in any type of antibody molecule where ADCC/ADCP/CDC effector function is desired to be reduced or even eliminated, or in molecules having similar antibody structures.

In one embodiment, the Fc mutant is substantially based on an IgG sequence. In one preferred embodiment, the Fc mutant is substantially based on a human IgG sequence. In another embodiment, the Fc mutant is substantially based on a human IgG1 sequence.

In another embodiment, the Fc mutant may also comprise other modifications, such as other modifications known in the art for reducing immunogenicity and improving stability, solubility, functions, and clinical benefits.

In one specific embodiment, the Fc mutant comprises the following sequence:
1) a sequence of amino acid positions 221-447 according to EU index in SEQ ID NO: 2, or an amino acid sequence having at least 90%, 91%, 92%, 93%, 94%, 95%, 96%, 97%, 98%, 99%, or even more identity to the sequence, or consisting of the sequence, or
2) a sequence of amino acid positions 221-447 according to EU index in SEQ ID NO: 3, or an amino acid sequence having at least 90%, 91%, 92%, 93%, 94%, 95%, 96%, 97%, 98%, 99%, or even more identity to the sequence, or consisting of the sequence, or
3) a sequence of amino acid positions 221-447 according to EU index in SEQ ID NO: 4, or an amino acid sequence having at least 90%, 91%, 92%, 93%, 94%, 95%, 96%, 97%, 98%, 99%, or even more identity to the sequence, or consisting of the sequence, or
4) a sequence of amino acid positions 221-447 according to EU index in SEQ ID NO: 5, or an amino acid sequence having at least 90%, 91%, 92%, 93%, 94%, 95%, 96%, 97%, 98%, 99%, or even more identity to the sequence, or consisting of the sequence, or
5) a sequence of amino acid positions 221-447 according to EU index in SEQ ID NO: 6, or an amino acid sequence having at least 90%, 91%, 92%, 93%, 94%, 95%, 96%, 97%, 98%, 99%, or even more identity to the sequence, or consisting of the sequence, or
6) a sequence of amino acid positions 221-447 according to EU index in SEQ ID NO: 7, or an amino acid sequence having at least 90%, 91%, 92%, 93%, 94%, 95%, 96%, 97%, 98%, 99%, or even more identity to the sequence, or consisting of the sequence, or
7) a sequence of amino acid positions 221-447 according to EU index in SEQ ID NO: 8, or an amino acid sequence having at least 90%, 91%, 92%, 93%, 94%, 95%, 96%, 97%, 98%, 99%, or even more identity to the sequence, or consisting of the sequence, or
8) a sequence of amino acid positions 221-447 according to EU index in SEQ ID NO: 11, or an amino acid sequence having at least 90%, 91%, 92%, 93%, 94%, 95%, 96%, 97%, 98%, 99%, or even more identity to the sequence, or consisting of the sequence, or
9) a sequence of amino acid positions 221-447 according to EU index in SEQ ID NO: 12, or an amino acid sequence having at least 90%, 91%, 92%, 93%, 94%, 95%, 96%, 97%, 98%, 99%, or even more identity to the sequence, or consisting of the sequence, or
10) a sequence of amino acid positions 221-447 according to EU index in SEQ ID NO: 13, or an amino acid sequence having at least 90%, 91%, 92%, 93%, 94%, 95%, 96%, 97%, 98%, 99%, or even more identity to the sequence, or consisting of the sequence, or
11) a sequence of amino acid positions 221-447 according to EU index in SEQ ID NO: 14, or an amino acid sequence having at least 90%, 91%, 92%, 93%, 94%, 95%, 96%, 97%, 98%, 99%, or even more identity to the sequence, or consisting of the sequence, or
12) a sequence of amino acid positions 221-447 according to EU index in SEQ ID NO: 15, or an amino acid sequence having at least 90%, 91%, 92%, 93%, 94%, 95%, 96%, 97%, 98%, 99%, or even more identity to the sequence, or consisting of the sequence, or
13) a sequence of amino acid positions 221-447 according to EU index in SEQ ID NO: 16, or an amino acid sequence having at least 90%, 91%, 92%, 93%, 94%, 95%, 96%, 97%, 98%, 99%, or even more identity to the sequence, or consisting of the sequence, or
14) a sequence of amino acid positions 221-447 according to EU index in SEQ ID NO: 17, or an amino acid sequence having at least 90%, 91%, 92%, 93%, 94%, 95%, 96%, 97%, 98%, 99%, or even more identity to the sequence, or consisting of the sequence, or
15) a sequence of amino acid positions 221-447 according to EU index in SEQ ID NO: 19, or an amino acid sequence having at least 90%, 91%, 92%, 93%, 94%, 95%, 96%, 97%, 98%, 99%, or even more identity to the sequence, or consisting of the sequence, or
16) a sequence of amino acid positions 221-447 according to EU index in SEQ ID NO: 20, or an amino acid sequence having at least 90%, 91%, 92%, 93%, 94%, 95%, 96%, 97%, 98%, 99%, or even more identity to the sequence, or consisting of the sequence, or
17) a sequence of amino acid positions 221-447 according to EU index in SEQ ID NO: 21, or an amino acid sequence having at least 90%, 91%, 92%, 93%, 94%, 95%, 96%, 97%, 98%, 99%, or even more identity to the sequence, or consisting of the sequence, or
18) a sequence of amino acid positions 221-447 according to EU index in SEQ ID NO: 22, or an amino acid sequence having at least 90%, 91%, 92%, 93%, 94%, 95%, 96%, 97%, 98%, 99%, or even more identity to the sequence, or consisting of the sequence, or
19) a sequence of amino acid positions 221-447 according to EU index in SEQ ID NO: 23, or an amino acid sequence having at least 90%, 91%, 92%, 93%, 94%, 95%, 96%, 97%, 98%, 99%, or even more identity to the sequence, or consisting of the sequence, or
20) a sequence of amino acid positions 221-447 according to EU index in SEQ ID NO: 24, or an amino acid sequence having at least 90%, 91%, 92%, 93%, 94%, 95%, 96%, 97%, 98%, 99%, or even more identity to the sequence, or consisting of the sequence, or
21) a sequence of amino acid positions 221-447 according to EU index in SEQ ID NO: 25, or an amino acid sequence having at least 90%, 91%, 92%, 93%, 94%, 95%, 96%, 97%, 98%, 99%, or even more identity to the sequence, or consisting of the sequence, or
22) a sequence of amino acid positions 221-447 according to EU index in SEQ ID NO: 27, or an amino acid sequence having at least 90%, 91%, 92%, 93%, 94%, 95%, 96%, 97%, 98%, 99%, or even more identity to the sequence, or consisting of the sequence, or
23) a sequence of amino acid positions 221-447 according to EU index in SEQ ID NO: 28, or an amino acid sequence having at least 90%, 91%, 92%, 93%, 94%, 95%, 96%, 97%, 98%, 99%, or even more identity to the sequence, or consisting of the sequence, or
24) a sequence of amino acid positions 221-447 according to EU index in SEQ ID NO: 29, or an amino acid sequence having at least 90%, 91%, 92%, 93%, 94%, 95%, 96%, 97%, 98%, 99%, or even more identity to the sequence, or consisting of the sequence, or
25) a sequence of amino acid positions 221-447 according to EU index in SEQ ID NO: 30, or an amino acid sequence having at least 90%, 91%, 92%, 93%, 94%, 95%, 96%, 97%, 98%, 99%, or even more identity to the sequence, or consisting of the sequence, or
26) a sequence of amino acid positions 221-447 according to EU index in SEQ ID NO: 31, or an amino acid sequence having at least 90%, 91%, 92%, 93%, 94%, 95%, 96%, 97%, 98%, 99%, or even more identity to the sequence, or consisting of the sequence, or
27) a sequence of amino acid positions 221-447 according to EU index in SEQ ID NO: 32, or an amino acid sequence having at least 90%, 91%, 92%, 93%, 94%, 95%, 96%, 97%, 98%, 99%, or even more identity to the sequence, or consisting of the sequence, or
28) a sequence of amino acid positions 221-447 according to EU index in SEQ ID NO: 33, or an amino acid sequence having at least 90%, 91%, 92%, 93%, 94%, 95%, 96%, 97%, 98%, 99%, or even more identity to the sequence, or consisting of the sequence.

In a second aspect, the present invention provides a polypeptide comprising the Fc mutant according to the first aspect. The polypeptide has reduced or eliminated ADCC and/or ADCP and/or CDC effector function compared to the polypeptide comprising a wild-type Fc region. Preferably, the polypeptide does not cause ADCC and/or ADCP and/or CDC effect. In one specific embodiment, the polypeptide simultaneously has a prolonged half-life. In one embodiment, the ADCC, CDC, and ADCP effector functions induced by the polypeptide comprising the Fc mutant according to the first aspect are reduced to at least 80%, 70%, 60%, 50%, 40%, 30%, 20%, 10%, 5%, 2%, or 1% of the ADCC, CDC, and ADCP effector functions induced by the polypeptide comprising the corresponding wild-type Fc, or completely eliminated.

In some embodiments, the polypeptide is an antibody molecule; preferably, the antibody molecule is an IgG antibody molecule. In another embodiment, the antibody molecule is a multispecific antibody (e.g., bispecific antibody), a humanized antibody, a chimeric antibody, or an antibody fusion. The antibody molecule comprising the Fc mutant obtained according to the present application has a reduced or even eliminated ability to bind to FcyR compared to the corresponding wild-type antibody molecule, and thus has reduced or eliminated ADCC and/or ADCP and/or CDC effector function.

In some embodiments, the polypeptide is a fusion protein comprising one or more fusion partners operably linked to the Fc mutant. The fusion partner can generally be any protein or small molecule, such as the variable region of any antibody, the target-binding region of a receptor, an adhesion molecule, a ligand, an enzyme, a cytokine, a chemokine, or some other proteins or protein domains. In one embodiment, the fusion protein is, for example, an immunoadhesin.

In one embodiment, the present application provides an IgG antibody comprising the Fc mutant, which comprises a deletion at amino acid position 329 (Δ329) according to EU index numbering as in Kabat. In another specific embodiment, the present application provides an IgG antibody comprising the Fc mutant, which comprises a deletion at amino acid positions 329 and 330 (Δ329 and Δ330) according to EU index numbering as in Kabat.

In one embodiment, the present application provides an IgG antibody comprising the Fc mutant, which comprises a substitution at amino acid positions 234 and/or 235 according to EU index numbering as in Kabat, wherein L, V, and F at position 234 and/or L at position 235 are substituted by A, V, L, and I, respectively. In one preferred embodiment, the IgG antibody comprises L234A + L235A, V234A, or F234A + L235A.

In one embodiment, the present application provides an IgG antibody comprising the Fc mutant, which comprises a deletion at amino acid position 329 and a substitution at position 330 according to EU index numbering as in Kabat, wherein position 330 is substituted by G, D, or Q. In one preferred embodiment, the IgG antibody comprises Δ329 + A330G or Δ329 + S330G.

In one embodiment, the present application provides an IgG antibody comprising the Fc mutant, which comprises a deletion at amino acid position 329 and a substitution at amino acid positions 234 and 235 according to EU index numbering as in Kabat, wherein L, V, and F at position 234 and/or L at position 235 are substituted by A, V, L, and I, respectively. In one preferred embodiment, the IgG antibody comprises L234A + L235A + Δ329, V234A + Δ329, or F234A + L235A + Δ329.

In one embodiment, the present application provides an IgG antibody comprising the Fc mutant, which comprises a deletion at amino acid positions 329 and 330 and a substitution at amino acid positions 234 and 235 according to EU index numbering as in Kabat, wherein L, V, and F at position 234 and/or L at position 235 are substituted by A, V, L, and I, respectively. In one preferred embodiment, the IgG antibody comprises L234A + L235A + Δ329 + Δ330, V234A + Δ329 + Δ330, or F234A + L235A + Δ329 + Δ330.

In one embodiment, the present application provides an IgG antibody comprising the Fc mutant, which comprises a deletion at amino acid position 329 and a substitution at amino acid positions 234, 235, and 330 according to EU index numbering as in Kabat, wherein L, V, and F at position 234 and/or L at position 235 are substituted by A, V, L, and I, respectively, and position 330 is substituted by G, D, or Q. In one preferred embodiment, the IgG antibody comprises L234A + L235A + A330G + Δ329, L234A + L235A + S330G + Δ329, V234A + L235A + A330G + Δ329, V234A + L235A + S330G + Δ329, F234A + L235A + S330G + Δ329, or F234A + L235A + A330G + Δ329.

In some embodiments, the IgG antibody provided herein is an IgG1 antibody, and the antibody has reduced or even eliminated ability to bind to FcyR compared to the corresponding wild-type antibody. In other embodiments, the IgG antibody provided herein is an IgG2, IgG3, or IgG4 antibody, and the antibody has reduced or even eliminated ability to bind to FcyR compared to the corresponding wild-type antibody. Preferably, the antibody provided herein retains the ability to bind to FcRn.

In some embodiments, the antibody molecule is an IgG antibody against claudin18.2.

In one specific embodiment, the IgG antibody comprising the Fc mutant provided herein comprises the following heavy chain and light chain:
1) comprising a heavy chain set forth in SEQ ID NO: 2 and a light chain set forth in SEQ ID NO: 9; or
2) comprising a heavy chain set forth in SEQ ID NO: 3 and a light chain set forth in SEQ ID NO: 9; or
3) comprising a heavy chain set forth in SEQ ID NO: 4 and a light chain set forth in SEQ ID NO: 9; or
4) comprising a heavy chain set forth in SEQ ID NO: 5 and a light chain set forth in SEQ ID NO: 9; or
5) comprising a heavy chain set forth in SEQ ID NO: 6 and a light chain set forth in SEQ ID NO: 9; or
6) comprising a heavy chain set forth in SEQ ID NO: 7 and a light chain set forth in SEQ ID NO: 9; or
7) comprising a heavy chain set forth in SEQ ID NO: 8 and a light chain set forth in SEQ ID NO: 9; or
8) comprising a heavy chain set forth in SEQ ID NO: 11 and a light chain set forth in SEQ ID NO: 9; or
9) comprising a heavy chain set forth in SEQ ID NO: 12 and a light chain set forth in SEQ ID NO: 9; or
10) comprising a heavy chain set forth in SEQ ID NO: 13 and a light chain set forth in SEQ ID NO: 9; or
11) comprising a heavy chain set forth in SEQ ID NO: 14 and a light chain set forth in SEQ ID NO: 9; or
12) comprising a heavy chain set forth in SEQ ID NO: 15 and a light chain set forth in SEQ ID NO: 9; or
13) comprising a heavy chain set forth in SEQ ID NO: 16 and a light chain set forth in SEQ ID NO: 9; or
14) comprising a heavy chain set forth in SEQ ID NO: 17 and a light chain set forth in SEQ ID NO: 9; or
15) comprising a heavy chain set forth in SEQ ID NO: 19 and a light chain set forth in SEQ ID NO: 9; or
16) comprising a heavy chain set forth in SEQ ID NO: 20 and a light chain set forth in SEQ ID NO: 9; or
17) comprising a heavy chain set forth in SEQ ID NO: 21 and a light chain set forth in SEQ ID NO: 9; or
18) comprising a heavy chain set forth in SEQ ID NO: 22 and a light chain set forth in SEQ ID NO: 9; or
19) comprising a heavy chain set forth in SEQ ID NO: 23 and a light chain set forth in SEQ ID NO: 9; or
20) comprising a heavy chain set forth in SEQ ID NO: 24 and a light chain set forth in SEQ ID NO: 9; or
21) comprising a heavy chain set forth in SEQ ID NO: 25 and a light chain set forth in SEQ ID NO: 9; or
22) comprising a heavy chain set forth in SEQ ID NO: 27 and a light chain set forth in SEQ ID NO: 34; or
23) comprising a heavy chain set forth in SEQ ID NO: 28 and a light chain set forth in SEQ ID NO: 34; or
24) comprising a heavy chain set forth in SEQ ID NO: 29 and a light chain set forth in SEQ ID NO: 34; or
25) comprising a heavy chain set forth in SEQ ID NO: 30 and a light chain set forth in SEQ ID NO: 34; or
26) comprising a heavy chain set forth in SEQ ID NO: 31 and a light chain set forth in SEQ ID NO: 34; or
27) comprising a heavy chain set forth in SEQ ID NO: 32 and a light chain set forth in SEQ ID NO: 34; or
28) comprising a heavy chain set forth in SEQ ID NO: 33 and a light chain set forth in SEQ ID NO: 34.

In another embodiment, the polypeptide (e.g., antibody) comprising the Fc mutant provided herein may also comprise other modifications in the Fc region, such as other modifications known in the art for reducing immunogenicity of a polypeptide (e.g., antibody), increasing half-life, and improving stability, solubility, functions, and clinical benefits.

In another embodiment, the antibody comprising the Fc mutant provided herein has a heavy chain variable region and a light chain variable region known in the art for different target antigens. Those skilled in the art can readily graft the heavy chain variable region and the light chain variable region known in the art onto the Fc mutant disclosed herein to obtain an antibody variant having desired properties (e.g., reduced or eliminated ADCC and/or ADCP and/or CDC effector function).

In a third aspect, the present invention provides a pharmaceutical composition comprising the polypeptide according to the second aspect and a pharmaceutically acceptable carrier. In one embodiment, the pharmaceutical composition comprises an antibody molecule carrying the Fc mutant described in the present invention. In one embodiment, the pharmaceutical composition comprises an IgG antibody carrying the Fc mutant described in the present invention. In another embodiment, the pharmaceutical composition comprises an IgG1 antibody, an IgG2 antibody, an IgG3 antibody, or an IgG4 antibody carrying the Fc mutant described in the present invention.

In a fourth aspect, the present invention provides a method for reducing or eliminating ADCC/ADCP/CDC effector function of an antibody, and retaining or even increasing half-life, according to the Fc region modification described in the present application. In one embodiment, ADCC/ADCP/CDC effector function of an antibody is reduced or eliminated by one or more modifications disclosed herein to the antibody Fc region in need of reducing or eliminating ADCC/ADCP/CDC effector function.

In a fifth aspect, the present invention provides use of the Fc mutant in preparing a drug. In one embodiment, the drug is for immunotherapy or adjuvant immunotherapy. In one embodiment, the drug has reduced or eliminated ADCC/ADCP/CDC effector function. In one specific embodiment, for example, the drug is a fusion protein comprising the Fc mutant of the present application, for example, an IL-2 Fc fusion protein. In another specific embodiment, for example, the drug is an antibody targeting an immune cell surface molecule comprising the Fc mutant of the present application, which has reduced or eliminated ADCC/ADCP/CDC effector function. In another embodiment, the drug is for treating a tumor in a subject. For example, in one embodiment, the drug activates immune cells of the patient without activating ADCC/ADCP/CDC effect and with a prolonged half-life, thereby achieving an anti-tumor effect.

In a sixth aspect, the present invention provides a method for treating a disease in a subject, comprising administering to the subject an effective amount of the pharmaceutical composition for the corresponding disease comprising the Fc mutant of the present application. In one embodiment, the disease is a disease requiring immunotherapy or adjuvant immunotherapy. For antibodies that target cell surface molecules, particularly antibodies on immune cells, it is advantageous to eliminate effector functions. In one embodiment, the present invention provides a method for treating a disease in a subject, comprising administering to the subject an effective amount of an antibody against an antigen on a corresponding immune cell comprising the Fc mutant of the present application.

In a seventh aspect, the present invention provides a kit comprising the Fc mutant disclosed herein, the polypeptide comprising the Fc mutant, or an immunoglobulin molecule comprising the Fc mutant. In one embodiment, the present invention provides a detection kit, which, by fusing functional molecules (such as enzymes, antigens, receptors, ligands, cytokines, and the like) with Fc, can improve the stability of fusion protein, and can also be applied to non-clinical fields such as flow cytometry, immunohistochemistry, *in vitro* activity detection, protein microarray detection, and the like.

The antibody molecule carrying a specific Fc mutation provided herein has reduced or eliminated ADCC, ADCP, and CDC effector functions, but still retains the ability to bind to FcRn, so that the antibody molecule can be used as an improved antibody mutant, which not only improves the curative effect of the antibody, but also ensures the safety, the stability and the low immunogenicity of the antibody. It has been surprisingly found that deletion of the proline residue at position 329 of the Fc region significantly reduces the binding of the Fc region to receptors FcyRIII, FcyRII, FcyRI, and C1q, thereby significantly reducing or eliminating ADCC, ADCP, and CDC activity. Furthermore, Pro329 of the Fc region and a combinatorial mutation, for example, selected from one or more of A330 deletion, A330G, L234A, and L235A result in a significant reduction of binding to the receptors FcyRIII, FcyRII, FcyRI, and C1q, thereby significantly reducing or eliminating ADCC, ADCP, and CDC activity.

### BRIEF DESCRIPTION OF THE DRAWINGS

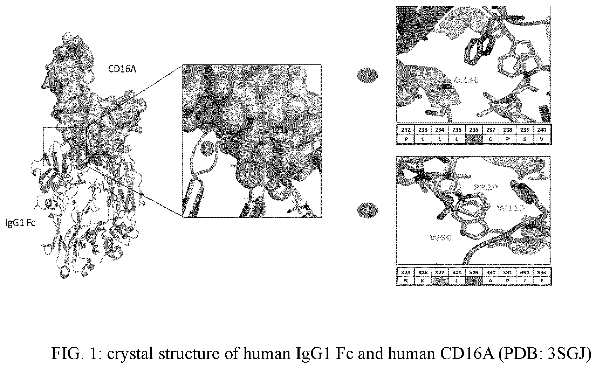

FIG. 1: crystal structure of human IgG1 Fc and human CD16A (PDB: 3SGJ).

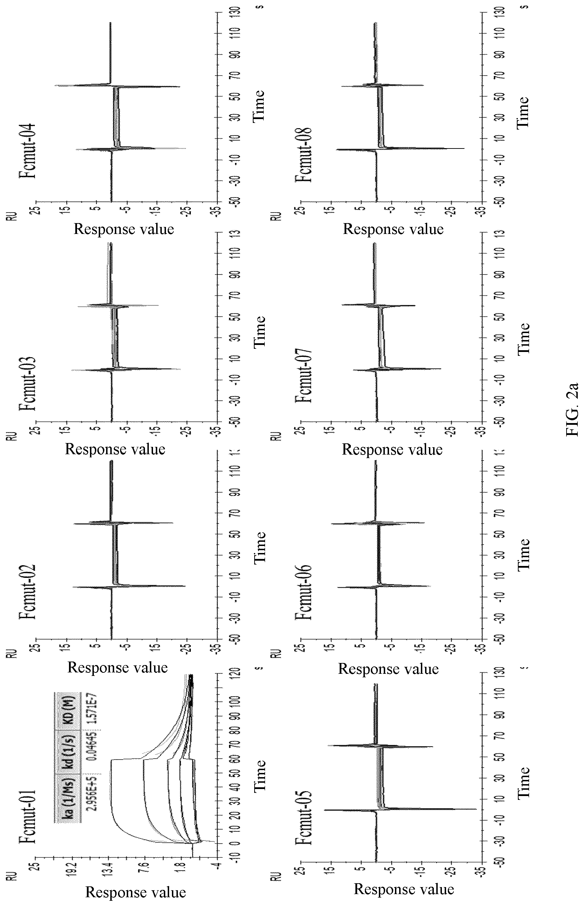

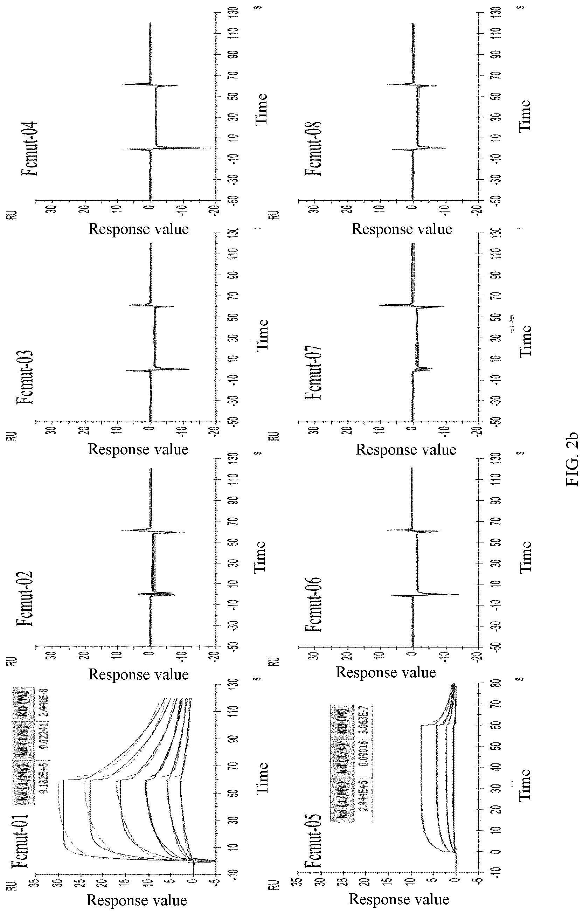

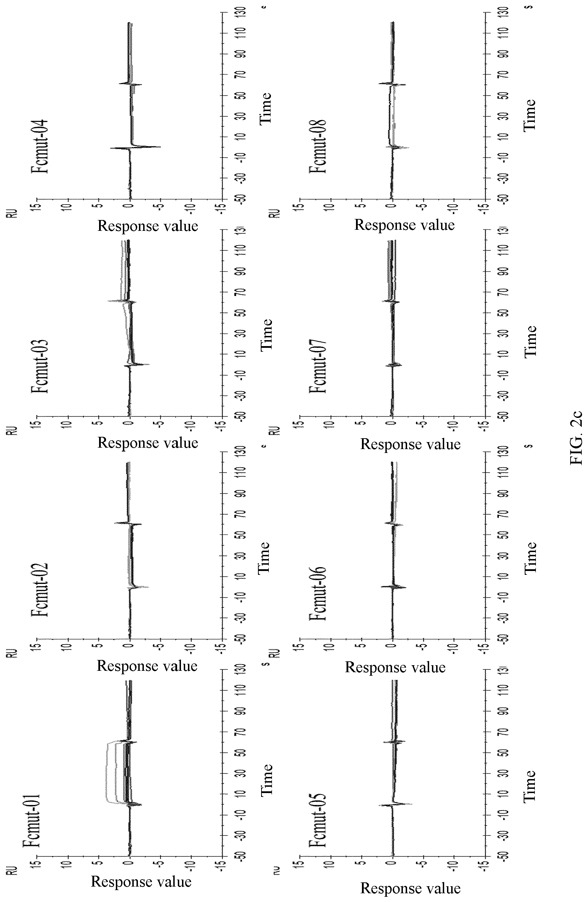

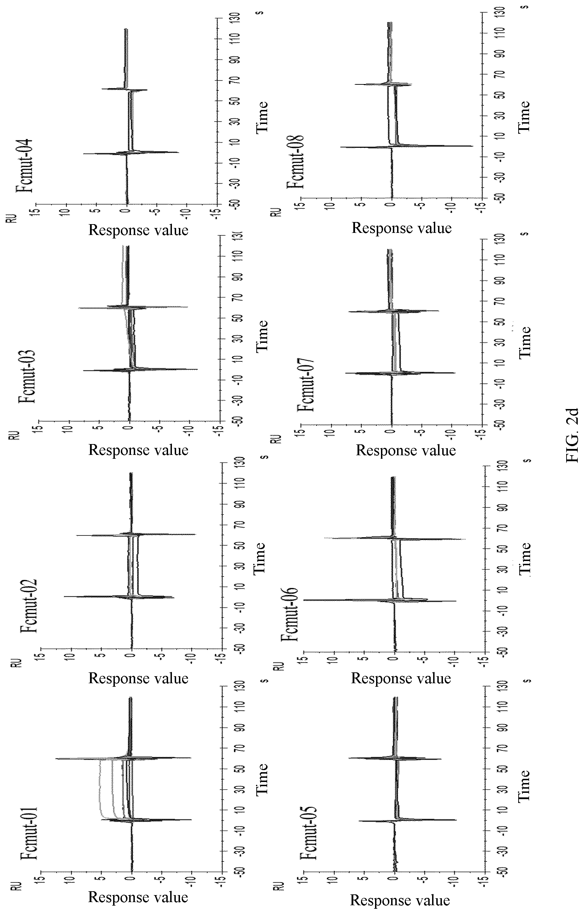

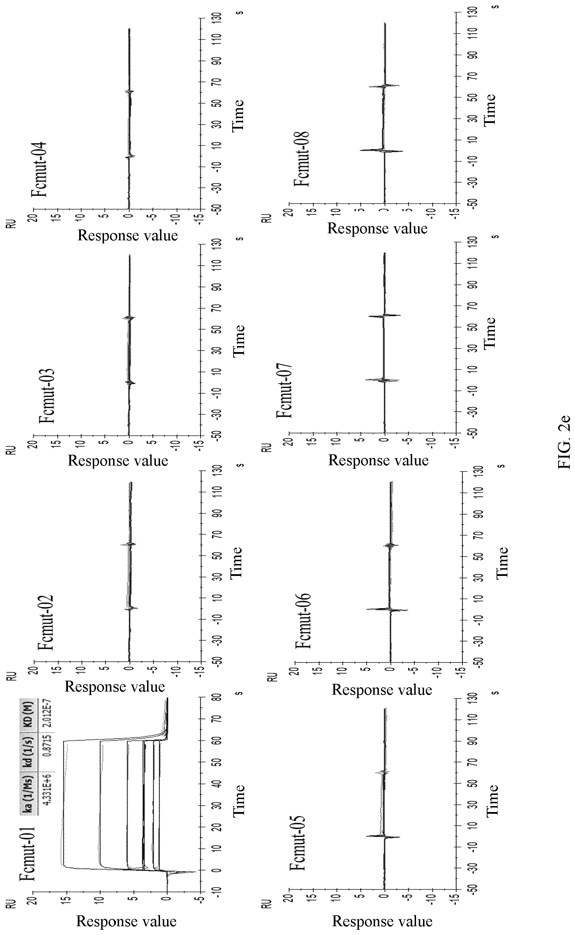

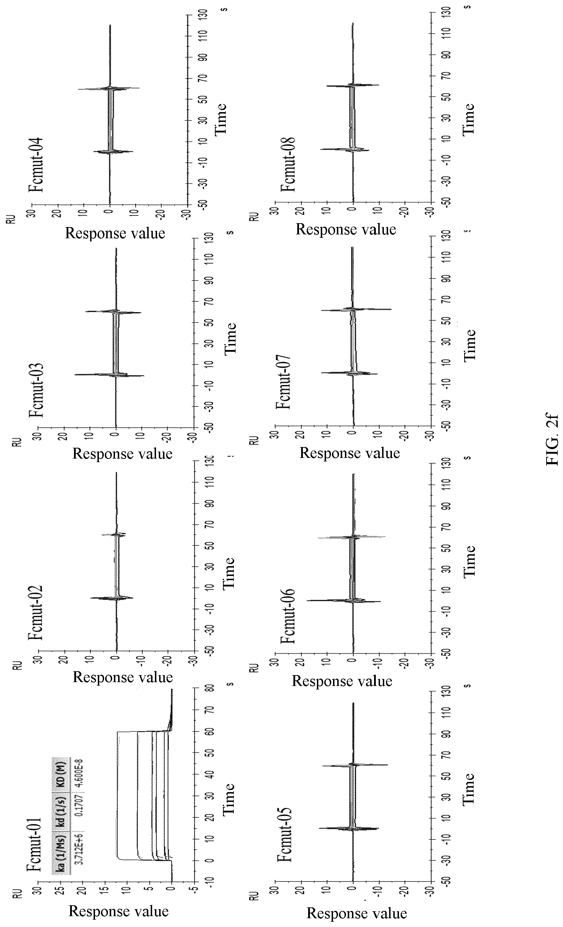

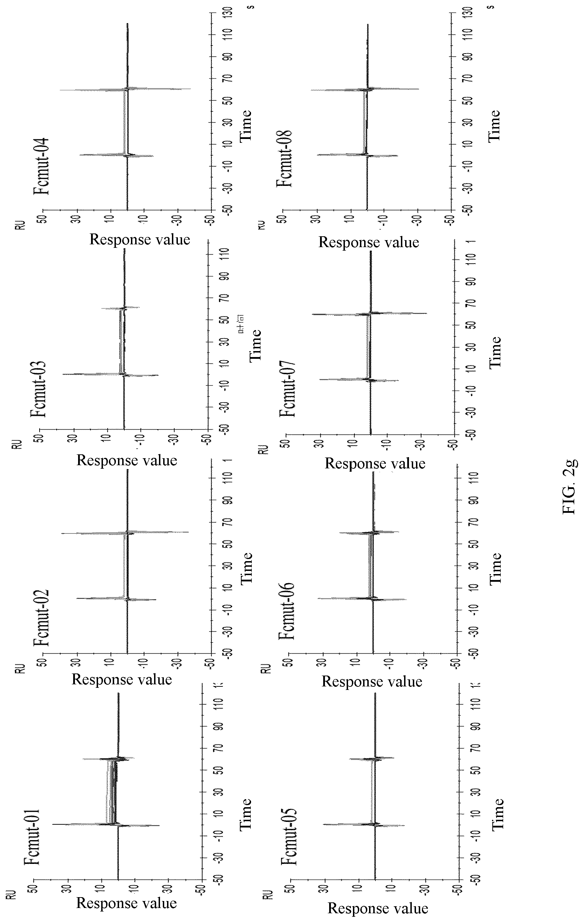

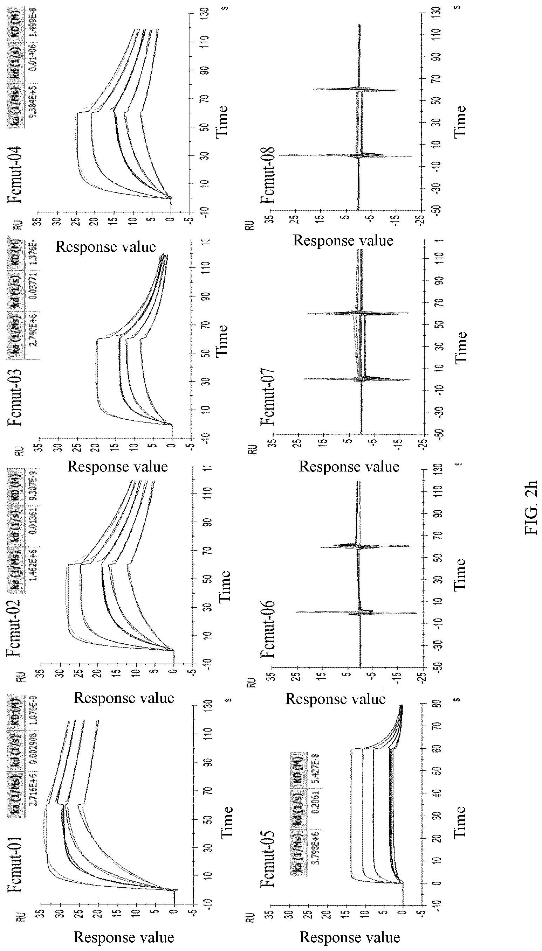

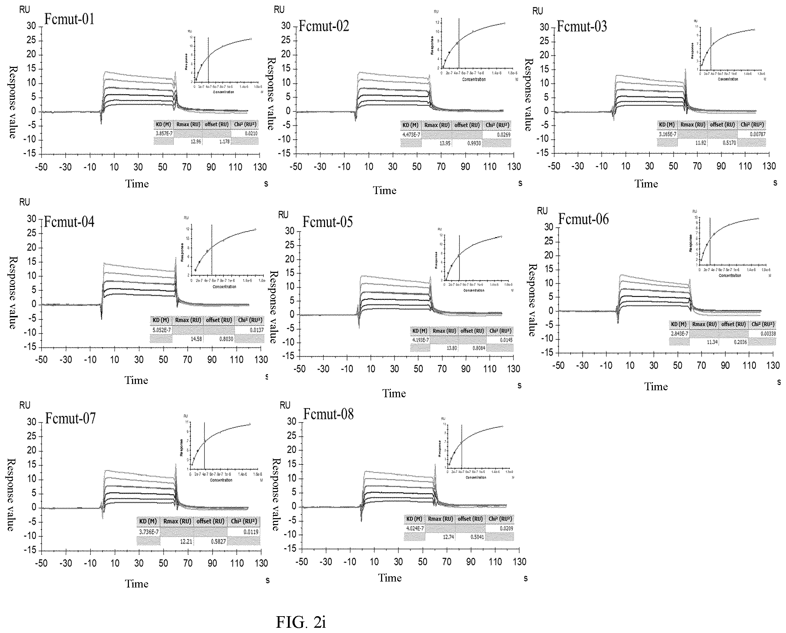

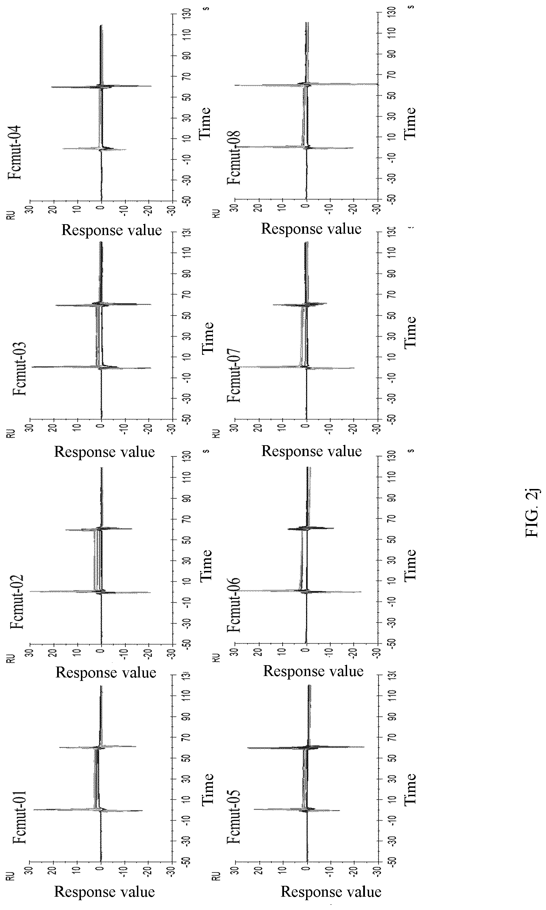

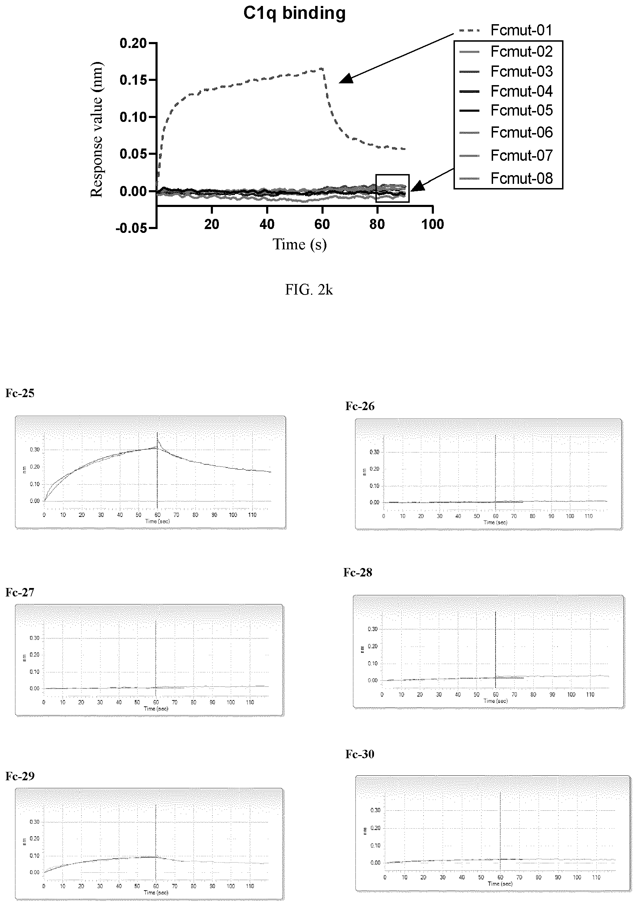

FIG. 2 shows the binding of the Fc mutants of the present invention to various FcyRs. FIG. 2a: binding curves of the Fc mutants to CD16A (F176) (pH 7.4). FIG. 2b: binding curves of the Fc mutants to CD16A (V176) (pH 7.4). FIG. 2c: binding curves of the Fc mutants to CD16B (NA1) (pH 7.4). FIG. 2d: binding curves of the Fc mutants to CD16B (NA2) (pH 7.4). FIG. 2e: binding curves of the Fc mutants to CD32A (H167) (pH 7.4). FIG. 2f: binding curves of the Fc mutants to CD32A (R167) (pH 7.4). FIG. 2g: binding curves of the Fc mutants to CD32B (pH 7.4). FIG. 2h: binding curves of the Fc mutants to CD64 (pH 7.4). FIG. 2i: binding curves of the Fc mutants to FcRn at pH 6.0. FIG. 2j: binding curves of the Fc mutants to FcRn at pH 7.0. FIG. 2k: binding and dissociation curves of the Fc mutants to C1q.

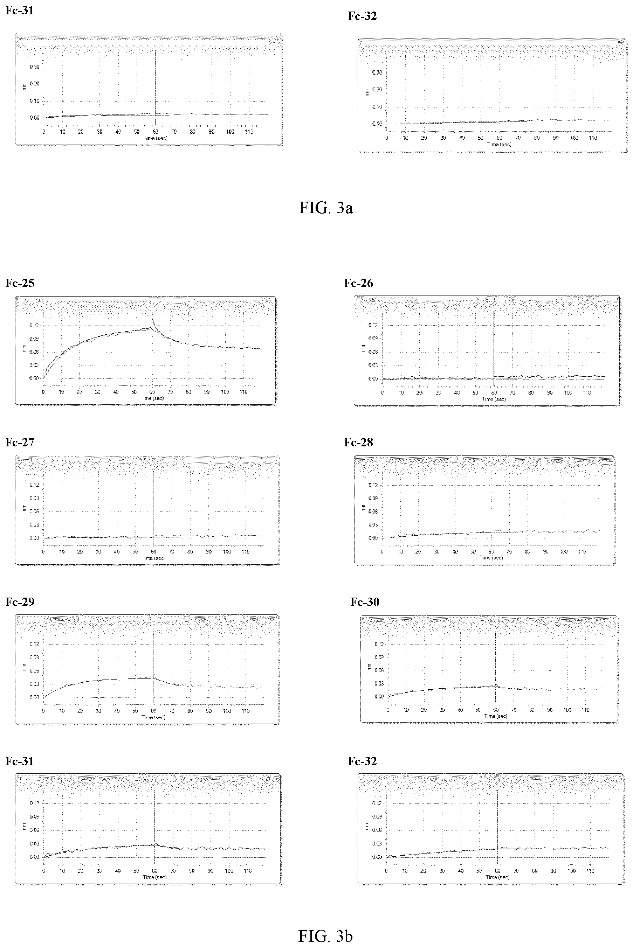

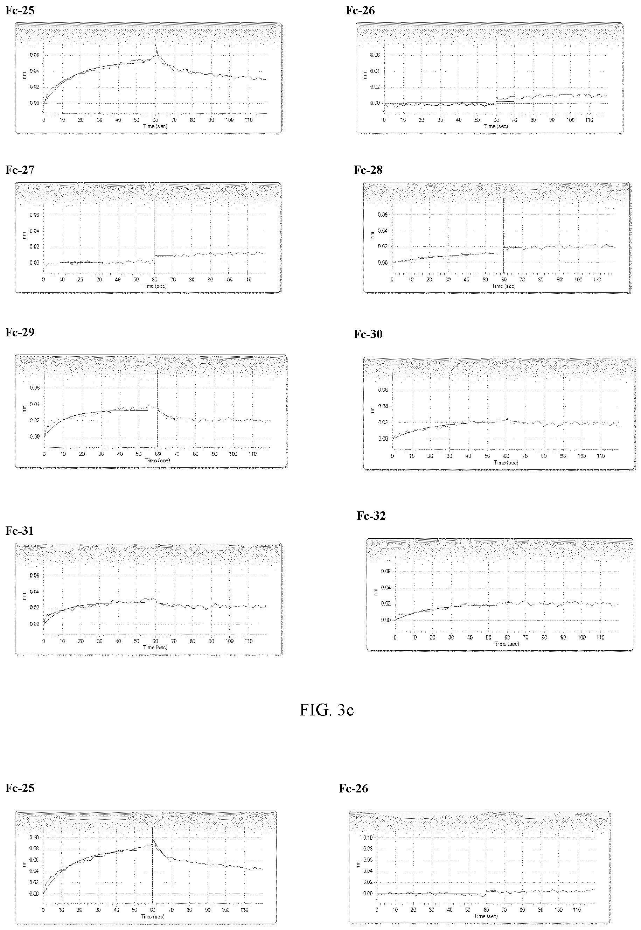

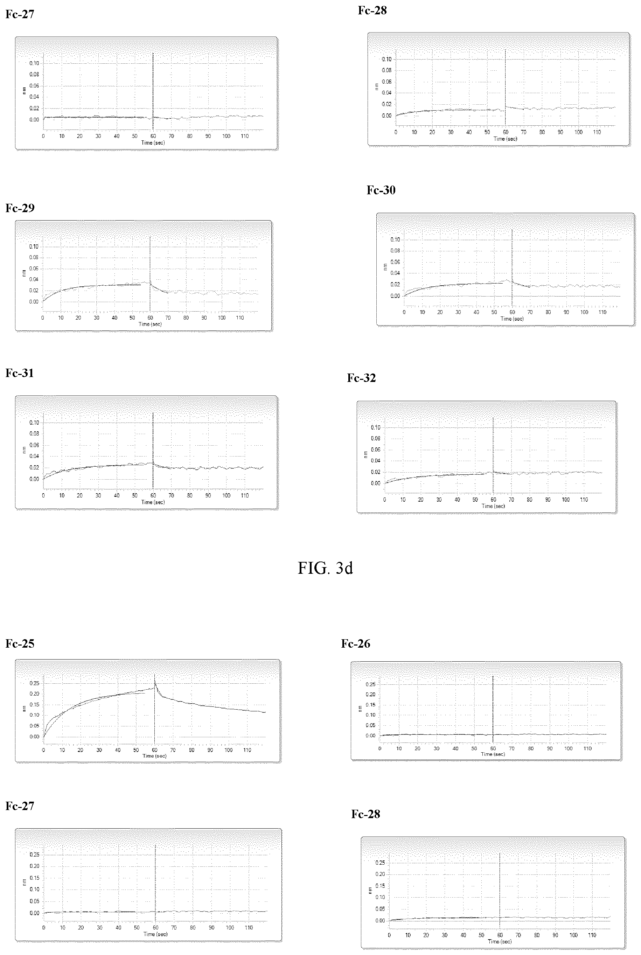

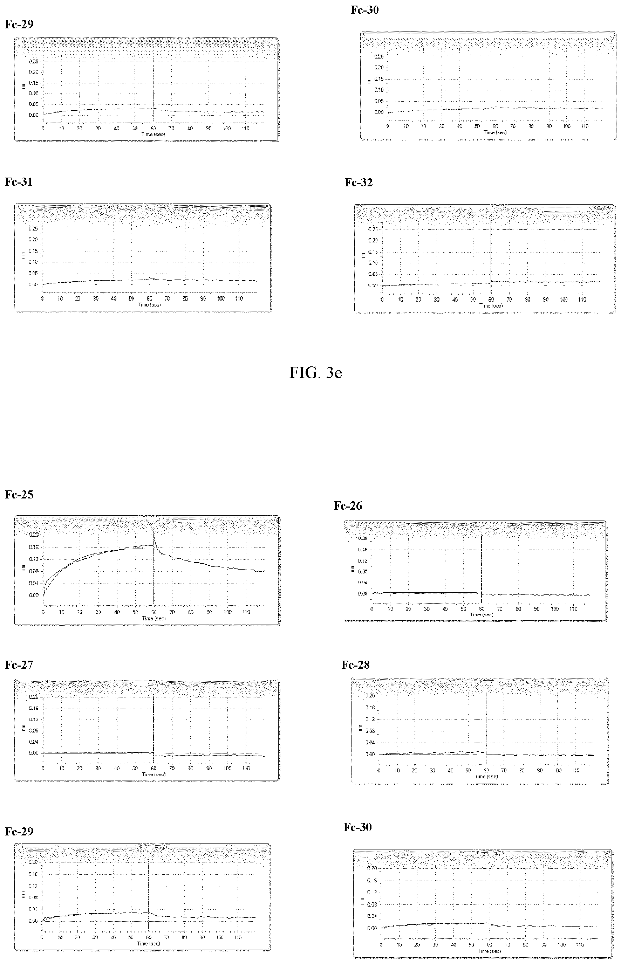

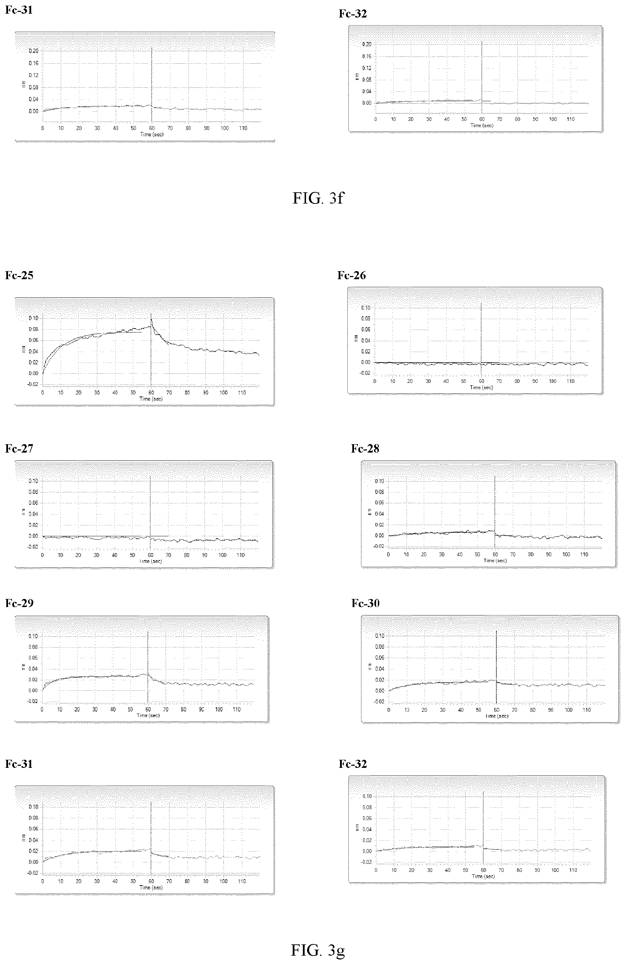

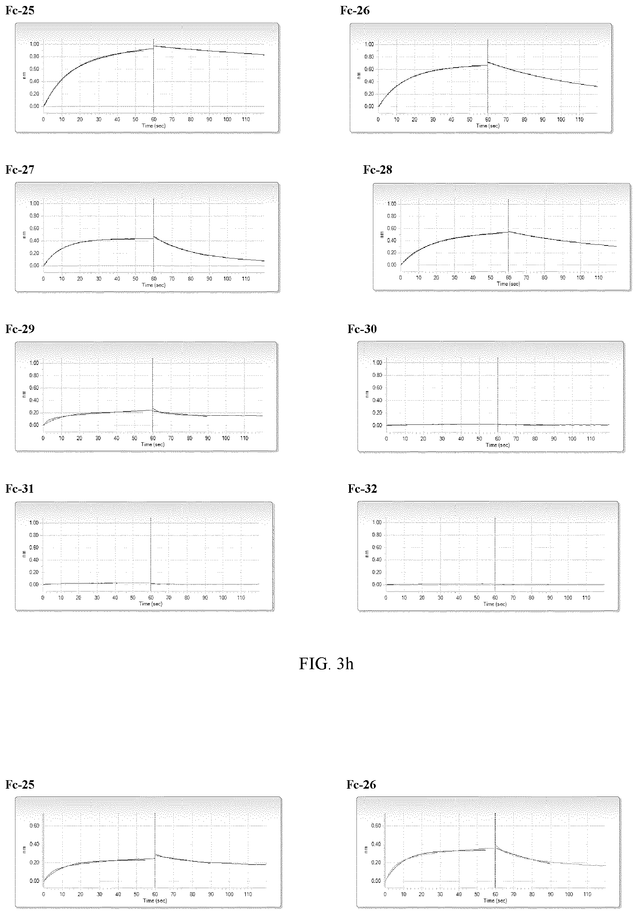

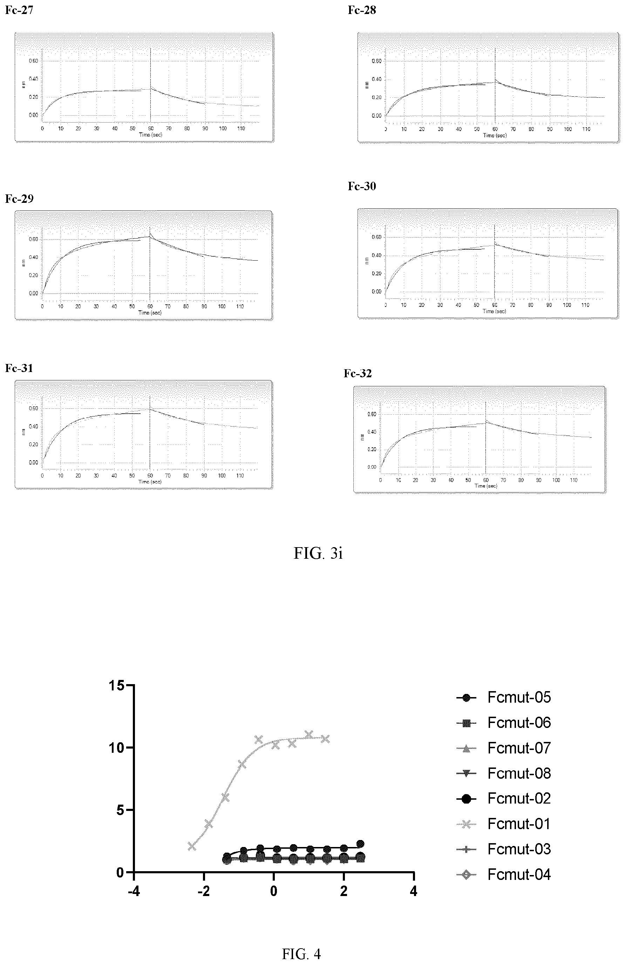

FIG. 3 shows the binding of the Fc mutants to various FcyRs as determined by bio-layer interferometry, wherein the solution comprises 200 nM of the antibody. FIG. 3a: binding curves of the Fc mutants to CD16A (V176) (pH 7.4). FIG. 3b: binding curves of the Fc mutants to CD16A (F176) (pH 7.4). FIG. 3c: binding curves of the Fc mutants to CD16B (NA1) (pH 7.4). FIG. 3d: binding curves of the Fc mutants to CD16B (NA2) (pH 7.4). FIG. 3e: binding curves of the Fc mutants to CD32A (H167) (pH 7.4). FIG. 3f: binding curves of the Fc mutants to CD32A (R167) (pH 7.4). FIG. 3g: binding curves of the Fc mutants to CD32B (pH 7.4). FIG. 3h: binding curves of the Fc mutants to CD64 (pH 7.4). FIG. 3i: binding curves of the Fc mutants to FcRn at pH 6.0.
FIG. 4 shows the ADCC activity of the antibodies.

### DETAILED DESCRIPTION

Unless otherwise indicated, conventional methods of chemistry, biochemistry, organic chemistry, molecular biology, microbiology, recombinant DNA techniques, genetics, immunology and cell biology that are known in the art will be employed for the implementation of the present invention. Descriptions of such methods can be found, for example, in Sambrook et al., Molecular Cloning: A Laboratory Manual (3rd edition, 2001); Sambrook et al., Molecular Cloning: A Laboratory Manual (2nd edition, 1989); Maniatis et al., Molecular Cloning: A Laboratory Manual (1982); Ausubel et. al., Current Protocols in Molecular Biology (John Wiley and Sons, updated in July 2008); Short Protocols in Molecular Biology: A Compendium of Methods from Current Protocols in Molecular Biology, Greene Pub. Associates and Wiley-Interscience; Glover, DNA Cloning: A Practical Approach, vol. I&II (IRL Press, Oxford, 1985); Anand, Techniques for the Analysis of Complex Genomes, (Academic Press, New York, 1992); Transcription and Translation (B. Hames & S. Higgins, Eds., 1984); Perbal, A Practical Guide to Molecular Cloning (1984); Harlow and Lane, Antibodies (Cold Spring Harbor Laboratory Press, Cold Spring Harbor, N.Y, 1998) Current Protocols in Immunology (Q. E. Coligan, A. M. Kruisbeek, D. H. Margufies, E. M. Shevach and W. Strober, eds., 1991); *Annual Review of Immunology*; and journals and monographs such as *Advances in Immunology.*

### Definitions

Before the present invention is described in detail below, it should be understood that the present invention is not limited to the particular methodology, protocols, and reagents described herein, as these may vary. It should also be understood that the terminology used herein is only intended to describe specific embodiments rather than limit the scope of the present invention, which will be limited only by the appended claims. Unless otherwise defined, any technical and scientific term used herein has the same meaning as commonly understood by those of ordinary skill in the art to which the present invention belongs.

For the purpose of explaining this specification, the following definitions will be used, and wherever appropriate, terms used in the singular form may also include the plural form, and vice versa. It should be understood that the terms used herein are for the purpose of describing specific embodiments only, and are not intended to be limiting.

The term "about" used in combination with a numerical value is intended to encompass the numerical values in a range from a lower limit less than the specified numerical value by 5% to an upper limit greater than the specified numerical value by 5%.

The term "and/or" means that when used to connect two or more options, it should be understood to refer to any one of the options or any two or more of the options.

The term "comprise" or "include" means that the described elements, integers or steps are included, but not to the exclusion of any other elements, integers or steps. The term "comprise" or "include" used herein, unless otherwise specified, also encompasses the situation where the entirety consists of the described elements, integers or steps. For example, when referring to an antibody variable region "comprising" a specific sequence, it is also intended to encompass an antibody variable region consisting of the specific sequence. The term "antibody" is used herein in the broadest sense and encompasses a variety of antibody structures, including but not limited to, a monoclonal antibody, a polyclonal antibody, a recombinant antibody, a humanized antibody, a chimeric antibody, a multispecific antibody (e.g., a bispecific antibody), a single-chain antibody, an intact antibody, or an antibody fragment thereof that exhibits the desired antigen-binding activity. An intact antibody will generally comprise at least two full-length heavy chains and two full-length light chains, but may comprise less chains in some cases, for example, natural antibodies in a camel may only comprise heavy chains.

As used herein, the term "binding" and "specific binding" mean that the binding effect of an antibody is selective for antigens and can be distinguished from unwanted or non-specific interactions. The ability of an antibody to bind to a particular antigen can be determined by an enzyme-linked immunosorbent assay (ELISA), surface plasmon resonance (SPR), or bio-layer interferometry (ForteBio), or a conventional binding assay known in the art. For example, if an antibody binds to BCMA or CD3 with a KD of about 1×10⁻⁷ or less, a KD of about 1×10⁻⁸ or less, a KD of about 1×10⁻⁹ or less, a KD of about 1×10⁻¹⁰ or less, or a KD of about 1×10⁻¹¹ or less in SPR, it is the antibody that "specifically binds to BCMA or CD3". However, the antibody that specifically binds to human BCMA or CD3 may have cross-reactivity with a BCMA or CD3 protein from other species. For example, an antibody specific to human BCMA or CD3, in some embodiments, can cross-react with cynomolgus monkey BCMA or CD3. A method for determining cross-reactivity includes the method described in examples and standard assays known in the art, such as biological optical interferometry or flow cytometry.

The terms "variable domain residue numbering of Kabat", "Kabat numbering system", or "amino acid position numbering according to Kabat" and variations thereof refer to the numbering system for antibody heavy chain variable domain or light chain variable domain editing according to Kabat et al. (see Kabat et al., Sequences of Proteins of Immunological Interest, 5th edition, Public Health Service, National Institutes of Health, Bethesda, Md. (1991)). The Kabat numbering system is generally applicable to residues in the variable domain of antibodies (approximately the light chain residues at positions 1-107 and the heavy chain residues at positions 1-113).

The terms "EU numbering system", "EU index as in Kabat", or "EU index" generally apply to residues in an immunoglobulin heavy chain constant region (see, e.g., Kabat et al. above). Unless otherwise indicated herein, residue numbering in antibody variable domains herein is according to the Kabat numbering system; residue numbering in antibody constant domains is according to the residue numbering of the EU numbering system. Antibody "effector function" refers to the biological activity attributed to the Fc region of an antibody (either the native sequence Fc region or the amino acid sequence variant Fc region) and varying with the antibody isotype. Examples of antibody effector functions include: complement-dependent cytotoxicity (CDC), antibody-dependent cell-mediated cytotoxicity (ADCC), antibody-dependent cellular phagocytosis (ADCP), cell surface receptor (such as B cell receptors) down-regulation, B-cell activation, and the like.

The term "effector cell" refers to a cell that expresses one or more FcRs and executes effector functions, such as a cell that expresses FcyRIIIA and executes effector function of ADCC. In one embodiment, a cell that mediates ADCC function is, for example, NK cells, peripheral blood mononuclear cells, monocytes, cytotoxic T cells, and neutrophils. Effector cells may be derived from natural environment, such as blood.

The term "antibody-dependent cell-mediated cytotoxicity" or "ADCC" refers to a cell-mediated immune response in which Fc receptors present on the surface of certain cytotoxic cells recognize bound antibodies on target cells, such that the cytotoxic cells can specifically bind to antigen-bearing target cells, and activate effector cells of the immune system to lyse the target cells. The classical ADCC effect is mediated by natural killer cells (NK). Macrophages, neutrophils, and eosinophils can also mediate the ADCC effect. For example, eosinophils can kill certain parasites through the ADCC effect.

The term "antibody-dependent cellular phagocytosis" or "ADCP" refers to a cellular response in which activation of macrophages is induced by binding of a target cell-binding antibody to FcyRIIIa on the macrophage surface, thereby internalizing the target cell and acidifying and degrading the target cell by a phagosome. ADCP may also be mediated by FcyRIIa and FcyRI, but the proportion is relatively small.

The complement system is part of the innate immune system that is composed of a series of proteins. The proteins of the complement system, called "complement", are represented by the abbreviations C1, C2, C3, and the like, and are a group of thermolabile proteins present in human or vertebrate serum and tissue fluids, which have enzymatic activity after activation. C1q is the first component of the complement-dependent cytotoxicity (CDC) pathway that is capable of binding to six antibodies, but binding to two IgG is sufficient to activate the complement cascade.

The term "complement-dependent cytotoxicity" or "CDC" refers to complement-involved cytotoxicity in which the Fc effector domain of an antibody that binds to a target activates a series of complement cascades, forming pores in the target cell membrane, and resulting in target cell death.

The term "Fc region" refers to the C-terminal region of an immunoglobulin heavy chain, including Fc regions of native sequences and variant Fc regions. The human IgG heavy chain Fc region is generally defined as the segment from the amino acid residue at position Cys226 or Pro230 to the carboxy terminus, and the C-terminus lysine residue at position 447 of the Fc region (according to the EU numbering system) may be present or absent. Thus, a composition of intact antibodies may comprise antibody populations with all K447 residues removed, antibody populations with no K447 residues removed, or antibody populations in which antibodies with K447 residues and antibodies without K447 residues are mixed.

In certain embodiments, the Fc region of an immunoglobulin comprises two constant domains, i.e., CH2 and CH3, and in other embodiments, the Fc region of an immunoglobulin comprises three constant domains, i.e., CH2, CH3, and CH4.

The binding of IgG to Fcy receptors or C1q is dependent on residues located in the hinge region and CH2 domains. Two regions of the CH2 domain are critical for the binding of FcyR to complement C1q, and have a unique sequence in IgG2 and IgG4. It has been shown that substitution of residues at positions 233-236 of human IgG1 and IgG2 and substitution of residues at positions 327, 330, and 331 of human IgG4 can significantly reduce ADCC and CDC activities (Armour et al., Eur. J. Immunol. 29(8), 1999, 2613-2624; Shields et al., J. biol. chem. 276(9), 2001, 6591-6604). Furthermore, Idusogie et al. show that alanine substitutions at various positions including K322 significantly reduce complement activation (Idusogie EE et al., J. Immunol 164(8), 2000, 4178-84).

"Functional Fc region" and similar terms are used interchangeably to refer to an Fc region having effector functions of a wild-type Fc region.

"Variant Fc region", "Fc mutant", "Fc region carrying a mutation", "mutant Fc region", "Fc region variant", "Fc variant", "mutated Fc region" and similar terms are used interchangeably to refer to an Fc region that comprises at least one amino acid modification and thus differs from the Fc region of the native sequence Fc region/wild-type Fc region.

In some embodiments, the variant Fc region comprises an amino acid sequence that differs from the amino acid sequence of the native sequence Fc region by one or more amino acid substitutions, deletions, or additions. In some embodiments, the variant Fc region has at least one amino acid deletion compared to the Fc region of a wild-type IgG. In some embodiments, the variant Fc region has at least one amino acid substitution compared to the Fc region of a wild-type IgG. In some embodiments, the variant Fc region has one or more amino acid substitutions and one or more amino acid deletions in the Fc region of the wild-type antibody. In some embodiments, the variant Fc region has at least one or two amino acid deletions in the Fc region described herein. In some embodiments, the variant Fc region has at least one, two, three, or more amino acid substitutions in the Fc region described herein. In some embodiments, the variant Fc region has at least one, two, three or more amino acid substitutions in the Fc region described herein and at least one or two deletions in the Fc region described herein. In some embodiments, the variant Fc region has at least about 80%, 90%, 95%, 96%, 97%, 98%, 99%, or more homology to a wild-type Fc region and/or a parent Fc region.

"Fc receptor" or "FcR" refers to a molecule that binds to the Fc region of an antibody. In some embodiments, the FcR is a native human FcR. In some embodiments, the FcR is a receptor that binds to an IgG antibody, i.e., FcyR, including three receptors, FcyRI (CD64), FcyRII (CD32), and FcγRIII (CD16), as well as allelic variants and alternative splice forms of the receptors. The FcγRII receptors include FcyRIIA and FcγRIIB, and the FcγRIII receptors include FcyRIIIA and FcγRIIIB.

The FcyR can be classified into activating receptors (FcyRI, FcyRIIA, FcyRIIC, FcγRIIIA, and FcyRIIIB, also known as CD64, CD32A, CD32C, CD16A, and CD16B) and inhibitory receptors (FcγRIIB, also known as CD32B) based on receptor function. The activating receptor comprises an immunoreceptor tyrosine-based activation motif (or ITAM) in the cytoplasmic domain thereof, which functions to transmit an activation signal and facilitate cell activation. The inhibitory receptor comprises an immunoreceptor tyrosine-based inhibitory motif (or ITIM) in the cytoplasmic domain thereof, which functions to inhibit cell activation. The effector functions of the activating FcyR are mainly ADCC, ADCP, and antigen presentation; the effector functions of the inhibitory FcyR are mainly inhibiting, cleaning, and the like. FcyRIIB is the only inhibitory FcyR expressed in humans and mice, and expression of FcyRIIB is associated with the decreased antibody curative effect in antibodies directly targeting tumors.

Different FcyRs have different cell expression profiles. For example, FcyRIIIA (CD16A) is expressed on macrophages, monocytes, natural killer cells (NK cells), and the like. The FcyRIIIA receptor is the only receptor expressed on NK cells, which can mediate ADCC function.

The term "FcR" also includes the neonatal receptor (FcRn). FcRn is an IgG antibody receptor located on the surface of cell membranes. FcRn is responsible for the transfer of maternal IgGs to the fetus and regulates the homeostasis of immunoglobulins *in vivo.* The FcRn can be combined with the Fc part of the IgG, prevents the IgG molecule from being cracked by lysosomes, can increase the half-life of the IgG *in vivo,* and participates in the *in vivo* transportation, maintenance, distribution, and metabolism processes of the IgG.

The IgG1-IgG4 subclasses have different abilities to bind to Fc receptors. IgG1 and IgG3 are universal ligands for different FcyRs, which bind to all FcyRs and have a strong ADCC effect. IgG2 or IgG4, also commonly referred to as the "inert" IgG subclass, is used to avoid immune activation effects. Indeed, many mAbs select IgG4 as the stem of the antibody to avoid ADCC effects. However, IgG2 and IgG4 are not completely "inert" and can bind to the activating forms of FcγRIIa-H131 and FcyRI, respectively, thereby initiating neutrophil activation.

Furthermore, in actual circumstances, there is a need to reduce ADCC effects caused by the IgG1 subclass, and in the prior art, "LALA" (L234A + L235A) mutation of IgG1, which can reduce the binding affinity of the Fc region of an antibody to FcyR by 100 times, has been widely used. The Fc mutant obtained by the present application, however, has lower binding affinity to FcyR compared to the "LALA" mutation, and thus more strongly reduces ADCC effects.

In the context of ADCC/ADCP/CDC, the expression "reduced ADCC and/or ADCP and/or CDC effector function" or "reduced ADCC/ADCP/CDC effector function" and similar expressions refer to a sufficiently high reduction in the value of ADCC and/or ADCP and/or CDC effector function caused by the Fc mutant of the present application, the polypeptide comprising the Fc mutant, or the like, as compared to the value of ADCC and/or ADCP and/or CDC effector function caused by the corresponding wild-type molecule, such that those skilled in the art will consider the reduction to be statistically significant within the corresponding biological context. For example, in certain embodiments, the reduction in the two values is, for example, greater than about 10%, greater than about 20%, greater than about 30%, greater than about 40%, greater than about 50%, greater than about 60%, greater than about 70%, greater than about 80%, greater than about 90%, or greater than about 100%, or even greater.

"Amino acid substitution" refers to the replacement of at least one amino acid residue present in a predetermined amino acid sequence by another, different "substituted" amino acid residue.

The term "conservative substitution" refers to a substitution of an amino acid by another amino acid of the same class, for example, the substitution of an acidic amino acid by another acidic amino acid, the substitution of a basic amino acid by another basic amino acid, or the substitution of a neutral amino acid by another neutral amino acid. Exemplary substitutions are shown in the table below:

| Original residue | Exemplary substitution | Preferred conservative substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |

| Original residue | Exemplary substitution | Preferred conservative substitution |
|---|---|---|
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

Amino acids can be grouped by common side chain properties: (1) hydrophobicity: Ile, Met, Ala, Val, Leu, and Ile; (2) neutral hydrophilicity: Cys, Ser, Thr, Asn, and Gln; (3) acidity: Asp and Glu; (4) alkalinity: His, Lys, and Arg; (5) residues affecting chain orientation: Gly and Pro; and (6) aromaticity: Trp, Tyr, and Phe. Non-conservative substitutions require the exchange of a member of one of these classes for another class.

"Amino acid deletion" refers to the removal of at least one amino acid residue from a predetermined amino acid sequence.

The terms "host cell", "host cell line" and "host cell culture" are used interchangeably and refer to cells into which exogenous nucleic acids are introduced, including progenies of such cells. Host cells include "transformants" and "transformed cells", which include original primary transformed cells and progenies derived therefrom, regardless of the number of passages. Progeny may not be exactly the same as parent cells in terms of nucleic acid content, and may comprise mutations. Mutant progenies having the same function or biological activities that are screened or selected from the initially transformed cells are included herein. The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needlema and Wunsch algorithm ((1970) J. Mol. Biol., 48:444-453; available at http://www.gcg.com) which has been integrated into the GAP program of the GCG software package, using the Blossom 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide acid sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5.

The percent identity between two amino acid sequences or nucleotide sequences can also be determined with PAM120 weighted remainder table, a gap length penalty of 12 and a gap penalty of 4, using the E. Meyers and W. Miller algorithm ((1989) CABIOS, 4:11-17) which has been incorporated into the ALIGN program (version 2.0).

### Target antigen of antibody

The antibody comprising the Fc mutant provided herein can target any antigen, including but not limited to proteins, subunits, domains, motifs, and/or epitopes belonging to the following target antigens. The target antigens are, for example, cytokines, membrane binding factors, enzymes, receptors, ligands, pathogens and toxins thereof, virus particles, tumor-associated factors, signaling pathway member molecules, or the like. Suitable antigens depend on the desired application. For anti-cancer therapy, it is desirable to have targets whose expression is restricted within the cancer cells. Some targets that have proven particularly suitable for antibody therapy are those with signaling functions. Other therapeutic antibodies exert their effect by inhibiting the binding between the receptor and the conjugate ligand thereof to block the signaling of the receptor.

Many antibodies that have been approved for clinical trials or are under development may benefit from the Fc mutant of the present invention. Thus, the variable regions of the antibodies can be integrated with the Fc mutant disclosed herein to form products with improved superior properties. In one embodiment, the Fc mutant disclosed herein may be integrated into humanized antibodies, affinity-matured antibodies, and engineered antibodies, e.g., fused to the heavy chain variable regions thereof.

### Additional modifications of Fc region

The Fc mutant provided herein, the fusion protein (e.g., antibody) comprising the Fc mutant, and the like can be further modified using a variety of methods already disclosed in the art, e.g., other modifications for reducing immunogenicity and improving stability, solubility, functions, and clinical benefits. Such modifications include, but are not limited to, for example, modifications at positions 252, 254, and 256, which can increase serum half-life.

### EXAMPLE

The following examples further illustrate the present invention. However, it should be understood that the examples are described by way of illustration rather than limitation, and various modifications may be made by those skilled in the art.

Unless otherwise indicated, conventional methods of chemistry, biochemistry, organic chemistry, molecular biology, microbiology, recombinant DNA techniques, genetics, immunology and cell biology that are known in the art will be employed for the implementation of the present invention.

### Example 1. Design of Fc Mutants

Given that the interaction of an antibody Fc region with an Fc receptor may have adverse effects, it is sometimes desirable in monoclonal antibody applications to eliminate effector functions of the Fc region (e.g., ADCC and CDC). For example, for the IgG1 subclass antibody, ADCC function is achieved primarily through the binding of the Fc region to FcyRIIIA. For the IgG1 subclass antibody requiring reduced ADCC and CDC functions, the Fc region thereof needs to be engineered to reduce binding to the Fcy receptor. Therefore, this example explores the key sites for the interaction of human IgG1 Fc with FcyR based on the cocrystal structure of the human IgG1 Fc and FcyR, and designs the corresponding Fc mutant to reduce the interaction thereof with FcyR.

The crystal structure (PDB: 3SGJ) of the human IgG1 Fc and human CD16A (FcγRIII) is shown in FIG. 1, wherein two segments of the Fc, P232-V240 and N325-E333, form the binding site for the CD16A epitope, wherein the amino acid P329 of the Fc interacts with the π-bond formed between amino acids W90 and W113 of the CD16A, which is the key amino acid in their binding interface. Based on the key segment and the amino acids, the inventors designed the Fc mutants as shown in Table 1 based on the intermolecular interaction interface.

In this example, an IgG1 monoclonal antibody (Fcmut-01) against human claudin18.2 protein obtained by internal screening of Innovent Biologies was used as an example for the relevant design and study. That is, a series of mutants having different Fc mutations (Fcmut-02 to Fcmut-024) shown in Table 1 were obtained based on Fcmut-01 as a parent and a control. Fcmut-25 was Herceptin (Genentech), and Fcmut-26 to Fcmut 32 were different engineerings on the Fc of the Herceptin.

**Table 1. Design of Fc Mutants**

| Molecule | Fc subtype | SEQ ID NO | | Fc mutation site (according to EU index in Kabat) | | | |
|---|---|---|---|---|---|---|---|
| | | Heavy chain | Light Chain | 234 | 235 | 329 | 330 |
| Fcmut-01 | IgG1-WT | SEQ ID NO :1 | SEQ ID NO :9 | L | L | P | A |
| Fcmut-02 | IgG1-mut | SEQ ID NO :2 | SEQ ID NO :9 | L | L | Deletion | A |
| Fcmut-03 | IgG1-mut | SEQ ID NO :3 | SEQ ID NO :9 | L | L | Deletion | G |
| Fcmut-04 | IgG1-mut | SEQ ID NO :4 | SEQ ID NO :9 | L | L | Deletion | Deletion |
| Fcmut-05 | IgG1-mut | SEQ ID NO :5 | SEQ ID NO :9 | A | A | P | A |
| Fcmut-06 | IgG1-mut | SEQ ID NO :6 | SEQ ID NO :9 | A | A | Deletion | A |
| Fcmut-07 | IgG1-mut | SEQ ID NO :7 | SEQ ID NO :9 | A | A | Deletion | G |
| Fcmut-08 | IgG1-mut | SEQ ID NO :8 | SEQ ID NO :9 | A | A | Deletion | Deletion |
| Fcmut-09 | IgG2-WT | SEQ ID NO :10 | SEQ ID NO :9 | V | A | P | A |
| Fcmut-10 | IgG2-mut | SEQ ID NO :11 | SEQ ID NO :9 | V | A | Deletion | A |
| Fcmut-11 | IgG2-mut | SEQ ID NO :12 | SEQ ID NO :9 | V | A | Deletion | G |
| Fcmut-12 | IgG2-mut | SEQ ID NO :13 | SEQ ID NO :9 | V | A | Deletion | Deletion |
| Fcmut-13 | IgG2-mut | SEQIDNO:14 | SEQ ID NO :9 | A | A | P | A |
| Fcmut-14 | IgG2-mut | SEQ ID NO :15 | SEQ ID NO :9 | A | A | Deletion | A |
| Fcmut-15 | IgG2-mut | SEQ ID NO :16 | SEQ ID NO :9 | A | A | Deletion | G |
| Fcmut-16 | IgG2-mut | SEQ ID NO :17 | SEQ ID NO :9 | A | A | Deletion | Deletion |
| Fcmut-17 | IgG4-S22 8P | SEQ ID NO :18 | SEQ ID NO :9 | F | L | P | S |
| Fcmut-18 | IgG4-mut | SEQ ID NO :19 | SEQ ID NO :9 | F | L | Deletion | S |
| Fcmut-19 | IgG4-mut | SEQ ID NO :20 | SEQ ID NO :9 | F | L | Deletion | G |
| Fcmut-20 | IgG4-mut | SEQ ID NO :21 | SEQ ID NO :9 | F | L | Deletion | Deletion |
| Fcmut-21 | IgG4-mut | SEQ ID NO :22 | SEQ ID NO :9 | A | A | P | S |
| Fcmut-22 | IgG4-mut | SEQ ID NO :23 | SEQ ID NO :9 | A | A | Deletion | S |
| Fcmut-23 | IgG4-mut | SEQ ID NO :24 | SEQ ID NO :9 | A | A | Deletion | G |
| Fcmut-24 | IgG4-mut | SEQ ID NO :25 | SEQ ID NO :9 | A | A | Deletion | Deletion |
| Fcmut-25 | IgG1-WT | SEQ ID NO :26 | SEQ ID NO :34 | L | L | P | A |
| Fcmut-26 | IgG1-mut | SEQ ID NO :27 | SEQ ID NO :34 | L | L | Deletion | A |
| Fcmut-27 | IgG1-mut | SEQ ID NO :28 | SEQ ID NO :34 | L | L | Deletion | G |
| Fcmut-28 | IgG1-mut | SEQ ID NO :29 | SEQ ID NO :34 | L | L | Deletion | Deletion |
| Fcmut-29 | IgG1-mut | SEQ ID NO :30 | SEQ ID NO :34 | A | A | P | A |
| Fcmut-30 | IgG1-mut | SEQ ID NO :31 | SEQ ID NO :34 | A | A | Deletion | A |
| Fcmut-31 | IgG1-mut | SEQ ID NO :32 | SEQ ID NO :34 | A | A | Deletion | G |
| Fcmut-32 | IgG1-mut | SEQ ID NO :33 | SEQ ID NO :34 | A | A | Deletion | Deletion |

### Example 2. Expression and Purification of Antibodies Carrying Fc Mutation

Plasmid construction: The nucleotide sequences of the heavy chain Fc mutation described above and the light chain were obtained according to the conventional experimental methods and cloned into pcDNA3.1 vectors to obtain each plasmid.

### Expression and purification of proteins

Preparation of transiently transfected plasmids: 1/10 (based on the transfection volume) of Opti-MEM^{™} reduced serum medium (Gibco, Catalog No. 31985-070) was added to the plasmid mixture (50 µg/50 mL, mass ratio of the heavy chain and light chain = 1:1). The Opti-MEM^{™} medium containing plasmids was filtered into a new 50-mL centrifuge tube, and filtered PEI (1 g/L, Polysciences) was added to the centrifugal tube (mass ratio of plasmid to PEI = 1:3). The mixture was mixed well and let stand for 20 min.

Cell transfection: The DNA/PEI mixture obtained above was poured gently to Expi293 cells (Gibco) and mixed well. The cells were transfected at 37 °C/8% CO₂ for 14 h, then 0.1% valproic acid sodium salt (VPA) (2.2 M, Sigma), 2.5% glucose (200 g/L, Sigma), and 2.5% Feed solution (1 g/L Phytone peptone + 1 g/L Difco Select Phytone) were added according to the volume of transfected cells, and the cells were incubated at 37 °C/8% CO₂ for 7d.

Product purification: The obtained cell culture broth was centrifuged at 4000 rpm for 50 min, and the supernatant was collected and purified by a pre-packed column Hitrap Mabselect Sure (GE, 11-0034-95). The specific procedures are as follows: The packed column was equilibrated with 5 column volumes of equilibration buffer (20 mM Tris, 150 mM NaCl, pH 7.2) before purification; the collected supernatant was loaded on the column, and then the column was washed with 10 column volumes of equilibration buffer to remove non-specific binding proteins; the column was washed with 5 column volumes of eluent buffer (100 mM sodium citrate, pH 3.5), and the eluate was collected. The eluate was adjusted to pH 6.0 with 2 M Tris and the concentration was measured to give purified antibody products.

The collected antibody products were buffer-exchanged into PBS (Gibco, 70011-044) by ultrafiltration concentration, and further separated and purified using superdex200 increase (GE, 10/300GL, 10245605). The elution peak of the monomer was collected, and the equilibration buffer and elution buffer for the column were PBS (Gibco, 70011-044).

### Example 3. Determination of Affinity of Fc Mutants for Fc Receptors

### I. Determination of affinity of antibodies carrying Fc mutants by SPR

The binding of the antibody carrying the Fc mutant obtained in the present invention to human FcyR was determined using surface plasmon resonance (SPR), wherein the equilibrium dissociation constant (KD) for each antibody mutant to FcyRI, FcγRIIa, FcγRIIb, FcγRIIIa, FcγRIIIb, and FcRn was specifically determined. Based on the principle of SPR, when a beam of polarized light entered the end face of a prism at a certain angle, surface plasma waves were generated at the interface between the prism and a gold film, causing free electrons in a metal film to generate resonance, namely surface plasmon resonance. When in analysis, a biomolecule recognition membrane was fixed on the surface of a sensing chip, then a sample to be detected flowed on the surface of the chip. If there were molecules capable of interacting with the biomolecule recognition membrane on the surface of the chip in the sample, the refractive index of the gold film surface was changed, finally causing changes in the SPR angle. The information such as the affinity and the kinetic constant of an analyte could be obtained by detecting the SPR angle changes.

In this example, the KD of the antibody carrying the Fc region mutation obtained in Example 2 and human FcyR was determined by Biacore (Cytiva, T200). The specific procedures were as follows: Each of FcyR and FcRn proteins (for information of each Fc receptor, see Table 2 below) containing a histidine tag was captured to the chip surface to which anti-histidine antibodies were coupled, and the binding and dissociation between the proteins on the chip surface and antibodies in the mobile phase were detected to obtain affinity and kinetic constants. The method comprises chip preparation and affinity detection. The assay procedure used 10×HBS-EP+ (BR-1006-69, Cytiva) diluted 10 times as an experimental buffer.

**Table 2. Information about receptor for detection**

| Receptor name | Tag | Cat# | Manufacturer |
|---|---|---|---|
| Human FcγRIIIB/CD16B (NA1) | his | CDB-H5227 | Acro |
| Human FcγRIIIB/CD16B (NA2) | his | CDB-H5222 | Acro |
| Human FcRn/FCGRT & B2M heterodimeric protein | His, Strep II | FCM-H5286 | Acro |
| Human FcγRIIIA/CD16A (V176) protein | his | CD8-H52H4 | Acro |
| Human FcγRIIIA/CD16A (F176) protein | his | CDA-H5220 | Acro |
| Human CD32A/FCGR2A protein (167his) | his | 10374-H27H1 | SINO |
| Human CD32A/FCGR2A protein (167Arg) | his | 10374-H27H | SINO |
| Human CD32B/FCGR2B protein | his | 10259-H08H | SINO |
| Human CD64/FCGR1A protein | his | 10256-H08S-B | SINO |

The specific procedures were as follows according to the manufacturer's instructions:
Chip preparation: The anti-histidine antibody in the histidine capture kit was coupled on the surface of a CM5 chip (29-1496-03, Cytiva) using the amino coupling kit (BR-1006-33, Cytiva) and the histidine capture kit (28995056, Cytiva), and the remaining activation sites were blocked by injection of 1 M ethanolamine after coupling.

Affinity assay: Each cycle included capture of the receptor, binding of a certain concentration of the antibody carrying the Fc region mutation of the present application, and regeneration of the chip. Each affinity assay cycle was performed on each antibody mutant solution after gradient dilution (0, 12.5, 25, 50, 100, 200, and 400 nM dilution gradient when binding to FcyRs; 0, 50, 100, 200, 400, 800, and 1600 nM dilution gradient when binding to FcRn) in an order from low to high concentrations. In each cycle, the antibody mutant solution flowed over the chip surface at a flow rate of 30 µL/min, with the binding time of 60 s and the dissociation time of 60 s. Finally, the chip was regenerated using 10 mM Glycine pH 1.5 (BR-1003-54, Cytiva). The obtained data were analyzed using Biacore T200 analysis software (version number 3.1) and using an analysis 1:1 binding or homeostasis analysis model to obtain the corresponding results.

Table 3 below shows the affinity data for each antibody mutant to each Fc receptor in the study, and FIG. 2 shows the fitted curve for the corresponding molecule.

From the results, it can be seen that: As a control antibody in the study, IgG1 wild-type Fcmut-01 antibody has high affinity for CD64 with the K_{D} value of 1.07E-09, binds to the two subtypes H167 and R167 of CD32A with the K_{D} values of 2.01E-07 and 4.60E-08, respectively, has affinity for the two subtypes F176 and V176 of CD16A of 1.57E-07 and 2.44E-08, respectively, and has weak binding to CD16B (NA1), CD16B (NA2), and CD32B.

Antibody molecules carrying the Fc mutation do not bind to CD16A (F176), CD16A (V176), CD16B (NA1), CD16B (NA2), CD32A (H167), CD32A (R167), and CD32B except the mutant Fcmut-05 which has a relatively weak affinity for CD16A (V176) (3.06E-07). With respect to CD64 which strongly binds to wild-type IgG1, none of the mutant Fcmut-06/07/08 binds to CD64, while the binding of Fcmut-02/03/04/05 to CD64 is also greatly reduced by 8.7 times, 12.9 times, 14.02 times, and 50.75 times, respectively, as compared to the control Fcmut-01. Furthermore, Fcmut-26 to Fcmut-32 also show similar results. Compared to Fcmut-25, antibody molecules carrying the Fc mutation do not bind to CD16A (F176), CD16A (V176), CD16B (NA1), CD16B (NA2), CD32A (H167), CD32A (R167), and CD32B except Fcmut-29.

F176 and V176 are the major genotypes of CD16A, and amino acid position 176 of CD16A is located in the binding region with Fc according to the crystal structure, thereby having an important effect on the affinity of CD16A for Fc. The Fc mutant obtained in the present application does not substantially interact with genotypes F176 and V176 of CD16A, so that it can be shown that the Fc mutant obtained in the present application will not substantially interact with all functional CD16A and thus will not cause ADCC effects. A protein molecule (e.g. an antibody) comprising the Fc mutant of the present application therefore has substantially no ADCC effector function.

The Fc region of an antibody is known to not bind to FcRn under neutral pH conditions but only under acidic conditions, which is the primary mechanism for a particularly long half-life of the antibody molecule. The control antibody Fcmut-01 is detected to have affinity for FcRn of 3.86E-07 at pH 6.0, but not to bind to FcRn at pH 7.0. The antibody molecule carrying the Fc mutation obtained in the study binds to FcRn in the same manner as the control antibody Fcmut-01 under the same conditions (FIGs. 2i and 2j), indicating that the Fc mutant obtained in the study does not affect the binding to FcRn.

FcRn is expressed on a variety of cells *in vivo,* and the binding of FcRn to the Fc region is pH dependent, the binding only around weak acidity pH 6.0, and no binding under neutral pH, thereby prolonging the half-life of the corresponding antibody. The experimental data of the present application show that the Fc region mutation carried by the antibody does not affect the binding of the antibody molecule to FcRn, so that the FcRn can still prolong the *in vivo* half-life of the antibody mutant of the present application.

The antibody mutant obtained in the present application does not bind to CD32B, which shows that the curative effect of the antibody can be enhanced by mutating the Fc region of the antibody.

The "LALA" mutation (Fcmut-05) widely used in the prior art reduces the binding affinity of the Fc region of an antibody to FcyR by 100 times. The Fc mutant obtained by the present application, however, has lower binding affinity to FcyR compared to the "LALA" mutation, and thus more strongly reduces ADCC effects. Wild-type IgG2 and IgG4 have a weak ADCC effect or have no ADCC effect, and therefore many antibody molecules for which ADCC effects are desired to be avoided select for production an Fc region of the IgG2 subtype or the IgG4 subtype. However, in some cases, it is still desirable to further reduce the ADCC effector function of the IgG2 subtype or the IgG4 subtype. Therefore, the applicants further investigated the binding of the corresponding sites of Fc regions of the IgG2 and IgG4 subtypes (see Table 1) after corresponding mutations to various FcRs. The data show that the IgG2 subtype or IgG4 subtype Fc mutant comprising the corresponding mutation of the present application reduces the binding to FcyR relative to wild-type Fc.

**Table 3. Binding constants of Fc mutants to Fc receptors**

| patent-ID | CD16A (F176) | CD16A (V176) | CD16B (NA1) | CD 16B (NA2) | CD32A (H167) | CD32A (R167) | CD32B | CD64 | FcRn (6.0) | FcRn (7.0) |
|---|---|---|---|---|---|---|---|---|---|---|
| Fcmut-01 | 1.57E-07 | 2.44E-08 | weak | weak | 2.01E-07 | 4.60E-08 | weak | 1.07E-09 | 3.86E-07 | N.B. |
| Fcmut-02 | N.B. | N.B. | N.B. | N.B. | N.B. | N.B. | N.B. | 9.31E-09 | 4.47E-07 | N.B. |
| Fcmut-03 | N.B. | N.B. | N.B. | N.B. | N.B. | N.B. | N.B. | 1.38E-08 | 3.17E-07 | N.B. |
| Fcmut-04 | N.B. | N.B. | N.B. | N.B. | N.B. | N.B. | N.B. | 1.50E-08 | 5.05E-07 | N.B. |
| Fcmut-05 | N.B. | 3.06E-07 | N.B. | N.B. | N.B. | N.B. | N.B. | 5.43E-08 | 4.19E-07 | N.B. |
| Fcmut-06 | N.B. | N.B. | N.B. | N.B. | N.B. | N.B. | N.B. | N.B. | 2.84E-07 | N.B. |
| Fcmut-07 | N.B. | N.B. | N.B. | N.B. | N.B. | N.B. | N.B. | N.B. | 3.74E-07 | N.B. |
| Fcmut-08 | N.B. | N.B. | N.B. | N.B. | N.B. | N.B. | N.B. | N.B. | 4.02E-07 | N.B. |

| | | | | | | | | | | |
|---|---|---|---|---|---|---|---|---|---|---|
| N. B.: non-bound | | | | | | | | | | |

### II. The binding of the Fc mutant of the present invention to Fc receptor as determined by bio-layer interferometry

The affinity (KD) for binding of the Fc mutant of the present invention to human Fc receptors was determined by bio-layer interferometry (BLI).

Half an hour before the experiment, an appropriate number of HIS1K sensors (18-5120, Sartorius) were taken according to the number of samples and soaked in the SD buffer (1× PBS, 0.1% BSA, 0.05% Tween-20), and the buffer used in the experiment was changed to 10 mM HEPS, 150 mM NaCl, 3 mM EDTA, 0.05% P20, pH 6.0 when FcRn was bound. The antibody was diluted to 200 nM and the Fc receptor (for sample information, see Table 2) was diluted to 100 nM.

200 µL of SD buffer, 200 µL of a labeled antibody solution, and 200 µL of Fc receptors were added to 96-well black polystyrene microplates (Greiner, 655209), respectively. Detection was conducted using Fortebio Octet Red96e, and the sensors were arranged according to the positions of the samples. The instrument settings were as follows: The operation procedures were baseline equilibration 120 s, Fc receptor immobilization 100 s, baseline equilibration 120 s, antibody binding 60 s, and dissociation 60 s at 1000 rpm and 30 °C. After the experiment was completed, KD values were analyzed using ForteBio Octet analysis software.

The results are shown in Table 4 below and FIG. 3. Compared to Fcmut-25, antibody molecules carrying the Fc mutation do not bind to CD16A (F176), CD16A (V176), CD16B (NA1), and CD16B (NA2) except Fcmut-29. The mutant Fcmut-26/27/28/29/30/31/32 do not bind to CD32A H167, CD32A R167, and CD32B. The mutant Fcmut-30/31/32 do not bind to CD64, and the binding of Fcmut-/26/27/28/29 to CD64 is also greatly reduced.

Fc-25 is an antibody that binds to HER2, the constant region of which is the wild-type IgG1 sequence. From the results, it can be seen that deletion of P329 reduces the binding of the molecule to the Fc gamma receptor, but the binding to FcRn (pH 6.0) is not affected.

**Table 4a. Mutants binding to CD16A V176 on HIS1K sensor**

| **Sample ID** | **Response** | **KD (M)** | **kon(1/Ms)** | **kdis(1/s)** |
|---|---|---|---|---|
| **Fc-25** | **0.297** | **9.43E-08** | **1.64E+05** | **1.54E-02** |
| **Fc-26** | **0.0029** | **N.B** | | |
| **Fc-27** | **0.007** | **N.B** | | |
| **Fc-28** | **0.0156** | **N.B** | | |
| **Fc-29** | **0.0909** | **1.55E-07** | **1.43E+05** | **2.22E-02** |
| **Fc-30** | **0.0211** | **N.B** | | |
| **Fc-31** | **0.0241** | **N.B** | | |
| **Fc-32** | **0.0141** | **N.B** | | |

**Table 4b. Mutants binding to CD16A F176 on HIS1K sensor**

| **Sample ID** | **Response** | **KD (M)** | **kon(1/Ms)** | **kdis(1/s)** |
|---|---|---|---|---|
| **Fc-25** | **0.1082** | **1.02E-07** | **1.98E+05** | **2.01E-02** |
| **Fc-26** | **0.0018** | **N.B** | | |
| **Fc-27** | **0.0023** | **N.B** | | |
| **Fc-28** | **0.0118** | **N.B** | | |
| **Fc-29** | **0.0439** | **2.06E-07** | **1.72E+05** | **3.54E-02** |
| **Fc-30** | **0.0232** | **N.B** | | |
| **Fc-31** | **0.0262** | **N.B** | | |
| **Fc-32** | **0.0166** | **N.B** | | |

**Table 4c. Mutants binding to CD16B NA1 on HIS1K sensor**

| **Sample ID** | **Response** | **KD (M)** | **kon(1/Ms)** | **kdis(1/s)** |
|---|---|---|---|---|
| **Fc-25** | **0.0531** | **6.45E-07** | **7.74E+04** | **4.99E-02** |
| **Fc-26** | **-0.002** | **N.B** | | |
| **Fc-27** | **0.0017** | **N.B** | | |
| **Fc-28** | **0.0115** | **N.B** | | |
| **Fc-29** | **0.0346** | **2.21E-07** | **2.35E+05** | **5.21E-02** |
| **Fc-30** | **0.0204** | **N.B** | | |
| **Fc-31** | **0.028** | **N.B** | | |
| **Fc-32** | **0.0184** | **N.B** | | |

**Table 4d. Mutants binding to CD16B NA2 on HIS1K sensor**

| **Sample ID** | **Response** | **KD (M)** | **kon(1/Ms)** | **kdis(1/s)** |
|---|---|---|---|---|
| **Fc-25** | **0.0801** | **1.05E-06** | **5.40E+04** | **5.68E-02** |
| **Fc-26** | **-0.0022** | **N.B** | | |
| **Fc-27** | **0.0037** | **N.B** | | |
| **Fc-28** | **0.0103** | **N.B** | | |
| **Fc-29** | **0.0331** | **4.82E-07** | **1.50E+05** | **7.23E-02** |
| **Fc-30** | **0.024** | **N.B** | | |
| **Fc-31** | **0.0269** | **N.B** | | |
| **Fc-32** | **0.0172** | **N.B** | | |

**Table 4e. Mutants binding to CD32A H167 on HIS1K sensor**

| **Sample ID** | **Response** | **KD (M)** | **kon(1/Ms)** | **kdis(1/s)** |
|---|---|---|---|---|
| **Fc-25** | **0.2124** | **6.12E-06** | **1.11E+04** | **6.81E-02** |
| **Fc-26** | **0.0058** | **N.B** | | |
| **Fc-27** | **0.0058** | **N.B** | | |
| **Fc-28** | **0.0142** | **N.B** | | |
| **Fc-29** | **0.0301** | **N.B** | | |
| **Fc-30** | **0.0171** | **N.B** | | |
| **Fc-31** | **0.0214** | **N.B** | | |
| **Fc-32** | **0.0097** | **N.B** | | |

**Table 4f. Mutants binding to CD32A R167 on HIS1K sensor**

| **Sample ID** | **Response** | **KD (M)** | **kon(1/Ms)** | **kdis(1/s)** |
|---|---|---|---|---|
| **Fc-25** | **0.1615** | **4.28E-06** | **1.66E+04** | **7.13E-02** |
| **Fc-26** | **0.0044** | **N.B** | | |
| **Fc-27** | **0.0028** | **N.B** | | |
| **Fc-28** | **0.0089** | **N.B** | | |
| **Fc-29** | **0.0293** | **N.B** | | |
| **Fc-30** | **0.0181** | **N.B** | | |
| **Fc-31** | **0.0199** | **N.B** | | |
| **Fc-32** | **0.0099** | **N.B** | | |

**Table 4g. Mutants binding to CD32B on HIS1K sensor**

| **Sample ID** | **Response** | **KD (M)** | **kon(1/Ms)** | **kdis(1/s)** |
|---|---|---|---|---|
| **Fc-25** | **0.0799** | **2.57E-07** | **2.18E+05** | **5.61E-02** |
| **Fc-26** | **-0.003** | **N.B** | | |
| **Fc-27** | **-0.0037** | **N.B** | | |
| **Fc-28** | **0.0066** | **N.B** | | |
| **Fc-29** | **0.0269** | **N.B** | | |
| **Fc-30** | **0.0171** | **N.B** | | |
| **Fc-31** | **0.0214** | **N.B** | | |
| **Fc-32** | **0.0083** | **N.B** | | |

**Table 4h. Mutants binding to CD64 on HIS1K sensor**

| **Sample ID** | **Response** | **KD (M)** | **kon(1/Ms)** | **kdis(1/s)** |
|---|---|---|---|---|
| **Fc-25** | **0.8932** | **9.48E-09** | **2.92E+05** | **2.77E-03** |
| **Fc-26** | **0.6477** | **5.33E-08** | **2.63E+05** | **1.40E-02** |
| **Fc-27** | **0.4326** | **1.13E-07** | **3.15E+05** | **3.56E-02** |
| **Fc-28** | **0.5144** | **4.66E-08** | **2.37E+05** | **1.10E-02** |
| **Fc-29** | **0.2287** | **4.45E-08** | **3.61E+05** | **1.61E-02** |
| **Fc-30** | **0.0202** | **N.B** | | |
| **Fc-31** | **0.0274** | **N.B** | | |
| **Fc-32** | **0.0162** | **N.B** | | |

**Table 4i. Mutants binding to FcRn on HIS1K sensor**

| **Sample ID** | **Response** | **KD (M)** | **kon(1/Ms)** | **kdis(1/s)** |
|---|---|---|---|---|
| **Fc-25** | **0.2392** | **2.84E-08** | **4.30E+05** | **1.22E-02** |
| **Fc-26** | **0.3488** | **5.09E-08** | **4.19E+05** | **2.13E-02** |
| **Fc-27** | **0.2855** | **6.23E-08** | **4.57E+05** | **2.84E-02** |
| **Fc-28** | **0.3545** | **4.55E-08** | **3.80E+05** | **1.73E-02** |
| **Fc-29** | **0.6114** | **3.28E-08** | **4.26E+05** | **1.40E-02** |
| **Fc-30** | **0.4914** | **2.21E-08** | **4.49E+05** | **9.93E-03** |
| **Fc-31** | **0.5716** | **2.58E-08** | **4.34E+05** | **1.12E-02** |
| **Fc-32** | **0.4803** | **2.39E-08** | **4.44E+05** | **1.06E-02** |

### Experimental Example 4. Affinity of Fc Mutant of Present Invention for C1q as Determined by Bio-Layer Interferometry

The strength of the affinity of the Fc antibody for C1q directly determines whether the antibody has CDC effector function, and therefore this example determines whether each Fc mutant has CDC effector function by detecting the affinity of the Fc mutant for C1q.

The affinity (KD) for binding of the antibodies of the present invention to human C1q was determined by bio-layer interferometry (BLI). A BLI affinity assay was conducted according to the existing method (Estep, P et al., High throughput solution based measurement of antibody-antigen affinity and epitope binding, MAbs, 2013.5(2): p. 270-8).

First, the EZ-LinkTM Sulfo-NHS-LC-Biotin (21327, Thermo Scientific) was mixed with the antibody in a molar ratio of 3:1, left to stand, and used for biotin labeling. The mixture was centrifuged to remove unlabeled biotin, and the antibody was exchanged into a PBS solution in equal volume.

Half an hour before the experiment, an appropriate number of SA sensors (Foretbio, 18-5019) were taken according to the number of samples and soaked in the SD buffer (1× PBS, 0.1% BSA, 0.05% Tween-20). The antibody was diluted to approximately 100 nM, and C1q (A099, Complement Technology) was diluted to 40 nM.

200 µL of SD buffer, 200 µL of the labeled antibody solution, and 200 µL of the C1q antigen were added to 96-well black polystyrene microplates (Greiner, 655209), respectively. Detection was conducted using Fortebio Octet Red96e, and the sensors were arranged according to the positions of the samples. The instrument settings were as follows: The operation procedures were Baseline, Loading -3 nm, Baseline, Association (Kon), and Dissociation (Kdis); the run time of each procedure was dependent on the rates of association and dissociation of samples; the rotation speed was 1000 rpm, and the temperature was 30 °C. After the experiment was completed, KD values were analyzed using ForteBio Octet analysis software, the coordinate axis data were derived, and Graphpad Prism 8 software was used for plotting.

The results are shown in Table 5 and FIG. 2k. Only Fcmut-01 can bind to C1q, the affinity is 2.02E-08, and none of Fcmut-2 to Fcmut-8 binds to C1q. The results show that the Fc mutant in the study can reduce or prevent the binding of Fc to C1q, and thus can reduce or eliminate CDC effector function of the antibody. Fcmut-10 to Fcmut-16 and Fcmut-18 to Fcmut-24 also do not bind to C1q, as compared with corresponding wild-type controls. Furthermore, Fcmut-26 to Fcmut-32 also do not bind to C1q, as compared with Fcmut-25.

**Table 5. Affinity of Fc mutants for C1q**

| Sample | Antigen | Response (nm) | KD (M) | Kon (1/Ms) | Kdis (1/s) |
|---|---|---|---|---|---|
| Fcmut-01 | C1q | 0.1582 | 2.02E-08 | 3.67E+06 | 7.41E-02 |
| Fcmut-02 | | 0.0007 | Non-bound | | |
| Fcmut-03 | | 0.0016 | Non-bound | | |
| Fcmut-04 | | -0.0027 | Non-bound | | |
| Fcmut-05 | | -0.0033 | Non-bound | | |
| Fcmut-06 | | -0.0035 | Non-bound | | |
| Fcmut-07 | | -0.0134 | Non-bound | | |
| Fcmut-08 | | 0.0004 | Non-bound | | |

### Experimental Example 5. Affinity of Antibody of Present Invention for Antigen as Determined by Bio-Layer Interferometry

The affinity assay was performed using the same BLI method as described in Example 4, except the following.

Half an hour before the experiment, an appropriate number of AHC sensors (Foretbio, 18-5060) were taken according to the number of samples and soaked in the SD buffer (1× PBS, 0.1% BSA, 0.05% Tween-20). The antibody and Claudin18.2 (cp0007, Genscript) were diluted to 100 nM, respectively.

200 µL of SD buffer, 200 µL of the antibody, and 200 µL of the Claudin18.2 antigen were added to 96-well black polystyrene microplates (Greiner, 655209), respectively. Detection was conducted using Fortebio Octet Red96e, and the sensors were arranged according to the positions of the samples. The instrument settings were as follows: The operation procedures were Baseline, Loading, Baseline, Association (Kon), and Dissociation (Kdis); the run time of each procedure was dependent on the rates of association and dissociation of samples; the rotation speed was 1000 rpm, and the temperature was 30 °C. After the experiment was completed, KD values were analyzed using ForteBio Octet analysis software.

The results are shown in Table 6. The KD values of Fcmut-02 to 08 and the antigen are in the same order of magnitude as the KD value of Fcmut-01 and the antigen, so that the specific mutation in the Fc region disclosed herein does not affect the affinity of the corresponding antibody for the antigen, further indicating that the antibody containing the Fc mutant of the present invention will retain the self-owned antigen affinity.

**Table 6. Affinity of antibodies for antigens**

| Sample | Antigen | Response (nm) | KD (M) | Kon (1/Ms) | Kdis (1/s) |
|---|---|---|---|---|---|
| Fcmut-01 | Claudin18.2 | 0.1493 | 3.32E-09 | 6.02E+04 | 2.00E-04 |
| Fcmut-02 | | 0.138 | 2.73E-09 | 7.32E+04 | 2.00E-04 |
| Fcmut-03 | | 0.1496 | 2.90E-09 | 6.90E+04 | 2.00E-04 |
| Fcmut-04 | | 0.1462 | 2.83E-09 | 7.07E+04 | 2.00E-04 |
| Fcmut-05 | | 0.153 | 3.17E-09 | 6.32E+04 | 2.00E-04 |
| Fcmut-06 | | 0.1444 | 2.44E-09 | 8.19E+04 | 2.00E-04 |
| Fcmut-07 | | 0.1538 | 2.47E-09 | 8.09E+04 | 2.00E-04 |
| Fcmut-08 | | 0.1517 | 2.41E-09 | 8.29E+04 | 2.00E-04 |

### Example 6. Antibody-Mediated ADCC Effect

One of the major effector functions mediated by FcyR is antibody-dependent cell-mediated cytotoxicity (ADCC), which is mediated by FcyRIIIA (CD16A) on NK cells and macrophages. After the affinity of the antibody carrying the Fc mutation obtained in the present application for Fc receptors is investigated in the previous example, this example continues to investigate the ADCC effector function of the antibody mutant. In this example, the Jurkat-ADCCNF-AT luciferase effector cell line (hereinafter referred to as ADCC effector cells) from Promega was used. The ADCC activity of the antibodies was detected by detecting the activation of NF-AT signal. The specific experimental process was as follows:
1) Preparation of cells
   Cells DANG-18.2 (CLS Cell Lines Service) overexpressing human claudin18.2 on the surface and ADCC effector cells were counted: Supernatants of cells DANG-18.2 and ADCC effector cells were removed by centrifugation. The cells were washed twice with a PBS solution and then resuspended in a detection medium (1640 medium with 5% low IgG serum (Gibco)). The concentration of ADCC effector cells was adjusted to 6 × 10⁶ cells/mL and the concentration of DANG-18.2 cells was adjusted to 1 × 10⁶ cells/mL.
2) Plating: The target cells DANG-18.2 were plated onto a 96-well plate at 25 µL/well.
3) Serial dilutions of the antibody of the present invention were added: The initial concentration of each antibody sample carrying the Fc region mutation and the control sample were as listed in Table 7, followed by three-fold dilution to obtain 10 dilution gradients, which were added to the well plates in sequence at 25 µL/well.

**Table 7. Initial concentration of antibody**

| Protein | Fcmut-01 | Fcmut-02 | Fcmut-03 | Fcmut-04 | Fcmut-05 | Fcmut-06 | Fcmut-07 | Fcmut-08 |
|---|---|---|---|---|---|---|---|---|
| Initial well protein concentration (µg/mL) | 30 | 300 | 300 | 300 | 300 | 300 | 300 | 300 |

| | | | | | | | | |
|---|---|---|---|---|---|---|---|---|
| 4) The ADCC effector cells were added to each well plate at 25 µL/well. 5) The cells were incubated in an incubator at 37 °C for 12 h. 6) The 96-well plate was taken out and stood at room temperature for 10 min, and 75 µL of thawed Luciferase assay reagent (Bio-Gio^{™} Luciferase assay reagent) was added to each well. The mixture was detected using a microplate reader, and the concentration-dependent curve was fitted with GraphPad software. | | | | | | | | |

As shown in FIG. 4, the control antibody Fcmut-01 has the Fc region of the wild-type IgG1 monoclonal antibody, which can effectively activate the NF-AT signal of ADCC effector cells by binding to the antigen on the target cell (DANG-18.2), thereby initiating the downstream signaling pathway of ADCC, indicating that the antibody has excellent ADCC killing ability. However, other antibodies carrying the Fc region mutations obtained in the present application exhibit very weak or nearly no ADCC effects, in particular: The mutant Fcmut-05 (carrying the L234A and L235A mutations) shows a very weak ADCC activity at a concentration 10 times greater than that of the control Fcmut-01, whereas the other mutants show no ADCC activity even at a high concentration 10 times greater than that of the control Fcmut-01, indicating that the ADCC effector function of the antibody molecule carrying the Fc region mutation obtained in the present application is substantially removed, and the result is consistent with the affinity data of each of the antibody molecules in Example 3 for CD16A.

According to the prior art, the LALA mutation of the Fc region can significantly reduce the ADCC effect of the antibody. The results of this example show that compared with the LALA mutation, the Fc mutant obtained in the present application has a larger reduction effect on the ADCC effect.

The antibody mutants of the present application obtained by mutation modifications (e.g., deletion and substitution) of one or more amino acids at positions 329, 330, 234, or 235 of the Fc region of the antibody substantially eliminate ADCC effector function, thereby indicating that the amino acid positions described above are critical for ADCC/ADCP effector function of the antibody. When the target antibody needs to avoid ADCC/ADCP effector function in real need, those skilled in the art will be able to select, based on the present disclosure, a corresponding modification on one or more of the amino acids at positions 329, 330, 234, or 235 of the Fc region to produce an actual technical effect. Specifically, deletion of the amino acid at position 329, deletion of the amino acid at positions 329-330, deletion of the amino acid at position 329 and substitution of the amino acid at position 320 of the Fc region of the antibody, and combined use of the modifications described above with the LALA modification (L234A + L235A) are contemplated to obtain an antibody molecule that eliminates the ADCC/ADCP effector function.

## Claims

1. An Fc mutant, comprising one or more amino acid modifications relative to a human wild-type Fc region, wherein the modification is selected from amino acid position 329, 234, 235, or 330, according to EU index numbering as in Kabat.

2. The Fc mutant according to claim 1, comprising the following modification:
1) a deletion at amino acid position 329 (Δ329) according to EU index numbering as in Kabat; or
2) a deletion at amino acid positions 329 and 330 (Δ329 and Δ330) according to EU index numbering as in Kabat; or
4) the following modification, according to EU index numbering as in Kabat: Δ329 + A330G or Δ329 + S330G; or
5) the following modification, according to EU index numbering as in Kabat: L234A + L235A + Δ329, V234A + Δ329, or F234A + L235A + Δ329; or
6) the following modification, according to EU index numbering as in Kabat: L234A + L235A + Δ329 + Δ330, V234A + Δ329 + Δ330, or F234A + L235A + Δ329 + Δ330; or
7) the following modification, according to EU index numbering as in Kabat: L234A + L235A + A330G + Δ329, L234A + L235A + S330G + Δ329, V234A + L235A + A330G + Δ329, V234A + L235A + S330G + Δ329, F234A + L235A + S330G + Δ329, or F234A + L235A + A330G + Δ329.

3. The Fc mutant according to claim 1 or 2, wherein the Fc mutant is a human IgG Fc mutant; preferably, the Fc mutant is an IgG1, IgG2, or IgG4 Fc mutant.

4. The Fc mutant according to any one of claims 1-3, comprising the following sequence:
1) a sequence of amino acid positions 221-447 according to EU index in SEQ ID NO: 2, or an amino acid sequence having at least 90%, 91%, 92%, 93%, 94%, 95%, 96%, 97%, 98%, 99%, or even more identity to the sequence, or consisting of the sequence, or
2) a sequence of amino acid positions 221-447 according to EU index in SEQ ID NO: 3, or an amino acid sequence having at least 90%, 91%, 92%, 93%, 94%, 95%, 96%, 97%, 98%, 99%, or even more identity to the sequence, or consisting of the sequence, or
3) a sequence of amino acid positions 221-447 according to EU index in SEQ ID NO: 4, or an amino acid sequence having at least 90%, 91%, 92%, 93%, 94%, 95%, 96%, 97%, 98%, 99%, or even more identity to the sequence, or consisting of the sequence, or
4) a sequence of amino acid positions 221-447 according to EU index in SEQ ID NO: 5, or an amino acid sequence having at least 90%, 91%, 92%, 93%, 94%, 95%, 96%, 97%, 98%, 99%, or even more identity to the sequence, or consisting of the sequence, or
5) a sequence of amino acid positions 221-447 according to EU index in SEQ ID NO: 6, or an amino acid sequence having at least 90%, 91%, 92%, 93%, 94%, 95%, 96%, 97%, 98%, 99%, or even more identity to the sequence, or consisting of the sequence, or
6) a sequence of amino acid positions 221-447 according to EU index in SEQ ID NO: 7, or an amino acid sequence having at least 90%, 91%, 92%, 93%, 94%, 95%, 96%, 97%, 98%, 99%, or even more identity to the sequence, or consisting of the sequence, or
7) a sequence of amino acid positions 221-447 according to EU index in SEQ ID NO: 8, or an amino acid sequence having at least 90%, 91%, 92%, 93%, 94%, 95%, 96%, 97%, 98%, 99%, or even more identity to the sequence, or consisting of the sequence, or
8) a sequence of amino acid positions 221-447 according to EU index in SEQ ID NO: 11, or an amino acid sequence having at least 90%, 91%, 92%, 93%, 94%, 95%, 96%, 97%, 98%, 99%, or even more identity to the sequence, or consisting of the sequence, or
9) a sequence of amino acid positions 221-447 according to EU index in SEQ ID NO: 12, or an amino acid sequence having at least 90%, 91%, 92%, 93%, 94%, 95%, 96%, 97%, 98%, 99%, or even more identity to the sequence, or consisting of the sequence, or
10) a sequence of amino acid positions 221-447 according to EU index in SEQ ID NO: 13, or an amino acid sequence having at least 90%, 91%, 92%, 93%, 94%, 95%, 96%, 97%, 98%, 99%, or even more identity to the sequence, or consisting of the sequence, or
11) a sequence of amino acid positions 221-447 according to EU index in SEQ ID NO: 14, or an amino acid sequence having at least 90%, 91%, 92%, 93%, 94%, 95%, 96%, 97%, 98%, 99%, or even more identity to the sequence, or consisting of the sequence, or
12) a sequence of amino acid positions 221-447 according to EU index in SEQ ID NO: 15, or an amino acid sequence having at least 90%, 91%, 92%, 93%, 94%, 95%, 96%, 97%, 98%, 99%, or even more identity to the sequence, or consisting of the sequence, or
13) a sequence of amino acid positions 221-447 according to EU index in SEQ ID NO: 16, or an amino acid sequence having at least 90%, 91%, 92%, 93%, 94%, 95%, 96%, 97%, 98%, 99%, or even more identity to the sequence, or consisting of the sequence, or
14) a sequence of amino acid positions 221-447 according to EU index in SEQ ID NO: 17, or an amino acid sequence having at least 90%, 91%, 92%, 93%, 94%, 95%, 96%, 97%, 98%, 99%, or even more identity to the sequence, or consisting of the sequence, or
15) a sequence of amino acid positions 221-447 according to EU index in SEQ ID NO: 19, or an amino acid sequence having at least 90%, 91%, 92%, 93%, 94%, 95%, 96%, 97%, 98%, 99%, or even more identity to the sequence, or consisting of the sequence, or
16) a sequence of amino acid positions 221-447 according to EU index in SEQ ID NO: 20, or an amino acid sequence having at least 90%, 91%, 92%, 93%, 94%, 95%, 96%, 97%, 98%, 99%, or even more identity to the sequence, or consisting of the sequence, or
17) a sequence of amino acid positions 221-447 according to EU index in SEQ ID NO: 21, or an amino acid sequence having at least 90%, 91%, 92%, 93%, 94%, 95%, 96%, 97%, 98%, 99%, or even more identity to the sequence, or consisting of the sequence, or
18) a sequence of amino acid positions 221-447 according to EU index in SEQ ID NO: 22, or an amino acid sequence having at least 90%, 91%, 92%, 93%, 94%, 95%, 96%, 97%, 98%, 99%, or even more identity to the sequence, or consisting of the sequence, or
19) a sequence of amino acid positions 221-447 according to EU index in SEQ ID NO: 23, or an amino acid sequence having at least 90%, 91%, 92%, 93%, 94%, 95%, 96%, 97%, 98%, 99%, or even more identity to the sequence, or consisting of the sequence, or
20) a sequence of amino acid positions 221-447 according to EU index in SEQ ID NO: 24, or an amino acid sequence having at least 90%, 91%, 92%, 93%, 94%, 95%, 96%, 97%, 98%, 99%, or even more identity to the sequence, or consisting of the sequence, or
21) a sequence of amino acid positions 221-447 according to EU index in SEQ ID NO: 25, or an amino acid sequence having at least 90%, 91%, 92%, 93%, 94%, 95%, 96%, 97%, 98%, 99%, or even more identity to the sequence, or consisting of the sequence, or
22) a sequence of amino acid positions 221-447 according to EU index in SEQ ID NO: 27, or an amino acid sequence having at least 90%, 91%, 92%, 93%, 94%, 95%, 96%, 97%, 98%, 99%, or even more identity to the sequence, or consisting of the sequence, or
23) a sequence of amino acid positions 221-447 according to EU index in SEQ ID NO: 28, or an amino acid sequence having at least 90%, 91%, 92%, 93%, 94%, 95%, 96%, 97%, 98%, 99%, or even more identity to the sequence, or consisting of the sequence, or
24) a sequence of amino acid positions 221-447 according to EU index in SEQ ID NO: 29, or an amino acid sequence having at least 90%, 91%, 92%, 93%, 94%, 95%, 96%, 97%, 98%, 99%, or even more identity to the sequence, or consisting of the sequence, or
25) a sequence of amino acid positions 221-447 according to EU index in SEQ ID NO: 30, or an amino acid sequence having at least 90%, 91%, 92%, 93%, 94%, 95%, 96%, 97%, 98%, 99%, or even more identity to the sequence, or consisting of the sequence, or
26) a sequence of amino acid positions 221-447 according to EU index in SEQ ID NO: 31, or an amino acid sequence having at least 90%, 91%, 92%, 93%, 94%, 95%, 96%, 97%, 98%, 99%, or even more identity to the sequence, or consisting of the sequence, or
27) a sequence of amino acid positions 221-447 according to EU index in SEQ ID NO: 32, or an amino acid sequence having at least 90%, 91%, 92%, 93%, 94%, 95%, 96%, 97%, 98%, 99%, or even more identity to the sequence, or consisting of the sequence, or
28) a sequence of amino acid positions 221-447 according to EU index in SEQ ID NO: 33, or an amino acid sequence having at least 90%, 91%, 92%, 93%, 94%, 95%, 96%, 97%, 98%, 99%, or even more identity to the sequence, or consisting of the sequence.

5. A polypeptide, comprising the Fc mutant according to any one of claims 1-4.

6. The polypeptide according to claim 5, wherein the polypeptide is an antibody molecule; preferably, the antibody molecule is an IgG antibody molecule.

7. The antibody molecule comprising the Fc mutant according to claim 6, comprising the following heavy chain and light chain:
1) comprising a heavy chain set forth in SEQ ID NO: 2 and a light chain set forth in SEQ ID NO: 9; or
2) comprising a heavy chain set forth in SEQ ID NO: 3 and a light chain set forth in SEQ ID NO: 9; or
3) comprising a heavy chain set forth in SEQ ID NO: 4 and a light chain set forth in SEQ ID NO: 9; or
4) comprising a heavy chain set forth in SEQ ID NO: 5 and a light chain set forth in SEQ ID NO: 9; or
5) comprising a heavy chain set forth in SEQ ID NO: 6 and a light chain set forth in SEQ ID NO: 9; or
6) comprising a heavy chain set forth in SEQ ID NO: 7 and a light chain set forth in SEQ ID NO: 9; or
7) comprising a heavy chain set forth in SEQ ID NO: 8 and a light chain set forth in SEQ ID NO: 9; or
8) comprising a heavy chain set forth in SEQ ID NO: 11 and a light chain set forth in SEQ ID NO: 9; or
9) comprising a heavy chain set forth in SEQ ID NO: 12 and a light chain set forth in SEQ ID NO: 9; or
10) comprising a heavy chain set forth in SEQ ID NO: 13 and a light chain set forth in SEQ ID NO: 9; or
11) comprising a heavy chain set forth in SEQ ID NO: 14 and a light chain set forth in SEQ ID NO: 9; or
12) comprising a heavy chain set forth in SEQ ID NO: 15 and a light chain set forth in SEQ ID NO: 9; or
13) comprising a heavy chain set forth in SEQ ID NO: 16 and a light chain set forth in SEQ ID NO: 9; or
14) comprising a heavy chain set forth in SEQ ID NO: 17 and a light chain set forth in SEQ ID NO: 9; or
15) comprising a heavy chain set forth in SEQ ID NO: 19 and a light chain set forth in SEQ ID NO: 9; or
16) comprising a heavy chain set forth in SEQ ID NO: 20 and a light chain set forth in SEQ ID NO: 9; or
17) comprising a heavy chain set forth in SEQ ID NO: 21 and a light chain set forth in SEQ ID NO: 9; or
18) comprising a heavy chain set forth in SEQ ID NO: 22 and a light chain set forth in SEQ ID NO: 9; or
19) comprising a heavy chain set forth in SEQ ID NO: 23 and a light chain set forth in SEQ ID NO: 9; or
20) comprising a heavy chain set forth in SEQ ID NO: 24 and a light chain set forth in SEQ ID NO: 9; or
21) comprising a heavy chain set forth in SEQ ID NO: 25 and a light chain set forth in SEQ ID NO: 9; or
22) comprising a heavy chain set forth in SEQ ID NO: 27 and a light chain set forth in SEQ ID NO: 34; or
23) comprising a heavy chain set forth in SEQ ID NO: 28 and a light chain set forth in SEQ ID NO: 34; or
24) comprising a heavy chain set forth in SEQ ID NO: 29 and a light chain set forth in SEQ ID NO: 34; or
25) comprising a heavy chain set forth in SEQ ID NO: 30 and a light chain set forth in SEQ ID NO: 34; or
26) comprising a heavy chain set forth in SEQ ID NO: 31 and a light chain set forth in SEQ ID NO: 34; or
27) comprising a heavy chain set forth in SEQ ID NO: 32 and a light chain set forth in SEQ ID NO: 34; or
28) comprising a heavy chain set forth in SEQ ID NO: 33 and a light chain set forth in SEQ ID NO: 34.

8. A pharmaceutical composition, comprising the Fc mutant according to any one of claims 1-4, or the polypeptide according to any one of claims 5-7, and a pharmaceutically acceptable carrier.

9. Use of the Fc mutant according to any one of claims 1-4 in reducing or eliminating ADCC, ADCP, or CDC effector functions.

10. A nucleic acid molecule encoding the Fc mutant according to any one of claims 1-4, or encoding the polypeptide according to any one of claims 5-7.

11. A vector, comprising the nucleic acid molecule according to claim 10.

12. A host cell, comprising the nucleic acid molecule according to claim 10 or the vector according to claim 11.

13. A method for treating a tumor in a subject, comprising administering an effective amount of the pharmaceutical composition according to claim 8 or the Fc mutant according to any one of claims 1-4 or the polypeptide according to any one of claims 5-7 to a subject in need thereof.

14. A kit, comprising the Fc mutant according to any one of claims 1-4 or the polypeptide according to any one of claims 5-7.

15. A method for preparing the Fc mutant according to any one of claims 1-4, or preparing the polypeptide according to any one of claims 5-7, comprising expressing the nucleic acid molecule according to claim 10 or expressing the vector according to claim 11 in the host cell according to claim 12 under appropriate conditions, wherein the method optionally further comprises recovering the expressed Fc mutant or polypeptide.
